# EUROPÄISCHE PATENTANMELDUNG

(11) **EP 0 790 312 A2**
(43) Veröffentlichungstag der Anmeldung: **20.08.1997**
(21) Anmeldenummer: 97101506.0
(22) Anmeldetag: 31.01.1997
(51) Int. Cl.: C12N 15/85, A61K 48/00

(54) **Zielzellspezifische Vektoren für die Einschleusung von Genen in Zellen, Arzneimittel enthaltend derartige Vektoren und deren Verwendung**

(30) Priorität: 13.02.1996 DE 19605279
(71) Anmelder: HOECHST AKTIENGESELLSCHAFT, 65929 Frankfurt am Main (DE)
(72) Erfinder: Sedlacek, Hans-Harald, Prof. Dr., 35041 Marburg (DE); Klenk, Hans-Dieter, Prof. Dr., 35440 Linden (DE); Kissel, Thomas, Prof. Dr., 35037 Marburg (DE); Müller, Rolf, Prof. Dr., 35037 Marburg (DE)

(57) **Zusammenfassung**

Offenbart werden zielzellspezifische Vektoren für die Einschleusung wenigstens eines Genes in Zellen eines Organismus, die folgende Komponenten umfassen:
a) einen nicht-viralen Träger für das einzuschleusende Gen,
b) einen Liganden, der spezifisch an die gewünschte Zielzelle binden kann,
c) ein Fusionsprotein für die Penetration des Vektors in das Zytoplasma der Zielzelle und
d) das einzuführende Gen.

Derartige Vektoren finden insbesondere bei der Gentherapie Verwendung.

SEQUENZPROTOKOLL

(1) ALLGEMEINE INFORMATION:

(i) ANMELDER:
(A) NAME: Hoechst Aktiengesellschaft
(B) STRASSE: -
(C) ORT: Frankfurt
(D) BUNDESLAND: -
(E) LAND: Deutschland
(F) POSTLEITZAHL: 65926
(G) TELEPHON: 069-305-3005
(H) TELEFAX: 069-35-7175
(I) TELEX: 4 1 234 700 ho d

(ii) ANMELDETITEL: Zielzellspezifische Vektoren fuer die
Einschleusung von Genen in Zellen, Arzneimittel enthaltend
derartige Vektoren und deren Verwendung

(iii) ANZAHL DER SEQUENZEN: 4

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 29 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..29

(xi) SEQUENZBESCHREIBUNG: SEQ ID NO: 1:

GAAGGATCCT GTGGGGCAAC AACACAATG 29

(2) INFORMATION ZU SEQ ID NO: 2:

(i) SEQUENZ CHARAKTERISTIKA:
(A) LÄNGE: 27 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..27

(xi) SEQUENZBESCHREIBUNG: SEQ ID NO: 2:

AAAAAGCTTC TTTCCCTTGT CACTAAA 27

(2) INFORMATION ZU SEQ ID NO: 3:

(i) SEQUENZ CHARAKTERISTIKA:
(A) LÄNGE: 18 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..18

(xi) SEQUENZBESCHREIBUNG: SEQ ID NO: 3:

CGGACTCTGA CCACTGAT 18

(2) INFORMATION ZU SEQ ID NO: 4:

(i) SEQUENZ CHARAKTERISTIKA:
(A) LÄNGE: 18 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..18

(xi) SEQUENZBESCHREIBUNG: SEQ ID NO: 4:

TCGTGGCAGA GGGAGTGT 18

## Beschreibung

Gegenstand der vorliegenden Erfindung sind zielzellspezifische Vektoren, die insbesondere in der Gentherapie Verwendung finden, Arzneimittel enthaltend derartige zielzellspezifische Vektoren und die Verwendung dieser Vektoren bei der Gentherapie. Die Gentherapie hat die Einschleusung fremder Gene in die Zellen eines Organismus zum Ziel, um defekte Gene in diesen Zellen auszuschalten, um defekte Gene durch intakte Gene zu ersetzen oder aber um von diesen Zellen ein Protein bilden zu lassen, das eine prophylaktische oder therapeutische Wirkung besitzt.

Aus dem Stand der Technik bekannt sind Vektoren für eukaryontische Systeme, die auf der Grundlage von Viren konstruiert wurden. Viren haben ein differenziertes System entwickelt, mit Hilfe dessen sie über Hüllproteine spezifisch an Zellen binden und nach der Aufnahme in Endosomen deren Membran penetrieren können. Demzufolge wurden Viren als Träger zur Einschleusung fremder Gene in die Zelle benutzt. Diese Technologie in ihren unterschiedlichen Variationen und die hierfür verwendeten Viren sind bereits ausführlich beschrieben worden (siehe die Übersichtsarbeiten von Hodgson, Bio/Technology 3, 222 (1995); Jolly, Cancer Gene Therapy 1, 51 (1994)).

Das Prinzip dieser Technologie ist, daß Teile des Virusgenes ersetzt werden durch das erwünschte Fremdgen, so daß ein viraler Vektor entsteht. Durch die Manipulation sind virale Vektoren im Regelfall nicht mehr vermehrungsfähig. Für die Vermehrung derartiger viraler Vektoren müssen jedoch alle Gene, welche die Virushüllproteine kodieren und die Expression dieser Virusgene regeln, vorhanden sein.

Es hat sich jedoch herausgestellt, daß virale Vektoren insbesondere beim Einsatz am Menschen problematisch sein können. Es besteht die Gefahr der Rekombination mit Wildtypviren gleicher Art, wodurch pathogene Viren entstehen könnten. Des weiteren können die Virushüllproteine Immunreaktionen im Empfänger auslösen. Da virale Vektoren in der Zelle den gleichen Infektionsweg wie die entsprechenden Wildviren einnehmen, besteht durch Integration der Fremdgene in die Wirtschromosomen die Gefahr der Mutationen der Wirtsgene (Aktivierung von ruhenden Genen, Zerstörung von aktiven Genen).

Ein weiterer Nachteil viraler Vektoren ist, daß durch die Geometrie der Viren ihre Aufnahmekapazität für fremde Gene beschränkt wird.

In Anbetracht dieser Einschränkungen und Gefahren von viralen Vektoren wurde schon früh versucht, virusunabhängige Methoden für die Einschleusung von Genen in Zellen zu finden. Das Prinzip dieser Methoden ist, die negativ geladene Zellmembran mit dem negativ geladenen Gen zu verschmelzen, so daß das Gen von der Zelle aufgenommen wird und durch die Endosomen- bzw. Lysosomenmembran in das Zytoplasma penetrieren kann. Neben physikalischen (Einschließen von Genpartikeln, osmotische, thermische oder elektrische Veränderungen der Zellmembran) oder chemischen (organische Lösungsmittel, Detergentien, Enzyme) Methoden zur Veränderung der Zellmembran wurden Träger für Gene entwickelt, die deren Verschmelzung mit der Zellmembran vermitteln. Hierzu gehören Liposomen, kationische Polypeptide, dendrimere Polymere oder kationische amphiphile Substanzen (Übersicht siehe bei Behr, Bioconjugate Chem. 5, 382 (1994); Afione et al., Clin. Pharmakokinet. 28, 181 (1995) und Felgner, Adv. Drug Delivery Rev. 5, 163 (1990)).

Größere Bedeutung für den Gentransfer haben synthetische kationische amphiphile Substanzen gewonnen wie beispielsweise Dioleoyloxypropyltrimethylammoniumbromid (DOTMA) im Gemisch mit Dioleoylphosphatidylethanolamin (DOPE) oder Lipopolyamin (siehe Behr, Bioconjugate Chem. 5, 382 (1994)).

Der Wirkungsmechanismus dieser kationischen amphiphilen Substanzen bzw. Substanzgemische ist, daß sie durch einen Überschuß an kationischer Ladung sowohl die negativ geladenen Gene komplexieren als auch an die anionische Zelloberfläche binden. Der amphiphile Charakter dieser Träger führt zur Fusion mit der Zellmembran.

Jedoch ist die zu erzielende Transfektionsrate immer noch deutlich geringer als mit viralen Vektoren. Desweiteren wird die kationische Überschußladung der Komplexe aus nicht viralen Trägern und DNA nach in vivo Applikation durch anionische biologische Substanzen (Proteine, Heparine etc.) neutralisiert, was zur Beeinträchtigung der Bindung an Zellen führt.

Die vorliegende Erfindung berücksichtigt die Überlegung, daß Zellen Gene über den Vorgang der Endozytose aufnehmen können. Dem Vorgang der Endozytose folgt regelmäßig der enzymatische Abbau der fremden Gene in den Endosomen bzw. Lysosomen. Nur solche Gene, welche sich diesem enzymatischen Abbau entziehen und durch die Membran der Endosomen/Lysosomen in das Zytoplasma und/oder in den Zellkern eindringen können, sind in der Lage, sich über die Transkription zu exprimieren. Bei den erfindungsgemäßen zielzellspezifischen Vektoren wird in vivo die lokale Konzentration der Vektoren an der Zielzelle erhöht, da die erfindungsgemäßen Vektoren mit zielzellspezifischen Liganden versehen sind.

Gegenstand der vorliegenden Erfindung sind daher zielzellspezifische Vektoren für die Einschleusung wenigstens eines Genes in Zellen eines Organismus umfassend folgende Komponenten:
a) einen nicht-viralen Träger für das einzuschleusende Gen,
b) einen Liganden, der spezifisch an die gewünschte Zielzelle binden kann,
c) ein Fusionsprotein für die Penetration des Vektors in das Zytoplasma der Zielzelle und
d) das einzuführende Gen.

Bei den erfindungsgemäßen Vektoren sind die einzelnen Komponenten des zielzellspezifischen Vektors miteinander kovalent und/oder durch adsorptive Bindung verbunden.

Bei den erfindungsgemäß verwendeten nicht-viralen Trägern (a) für das Gen handelt es sich bevorzugt um Proteine, Polypeptide, Polysaccharide, Phospholipide, kationische Lipide, Glykoproteine, Lipoproteine oder Lipopolyamine, die durch Einführung positiv geladener Seitengruppen kationisiert sein können, wobei die Bindung zwischen nicht-viralem Träger und positiv geladener Seitenkette durch adsorptive oder kovalente Bindung bewirkt wird. Durch eine zusätzliche adsorptive oder kovalente Bindung von lipophilen Seitengruppen kann der Träger des weiteren amphiphile Eigenschaften erlangen.

In einer besonders bevorzugten Ausführungsform handelt es sich bei dem nicht-viralen Träger (a) um Albumin oder Xylan.

Der erfindungsgemäß eingesetzte Ligand (b) kann spezifisch an die äußere Membran tierischer oder menschlicher Zellen binden.

In einer bevorzugten Ausführungsform kann der Ligand (b) spezifisch an Endothelzellen binden und ist ausgewählt aus der Gruppe bestehend aus monoklonalen Antikörpern oder deren Fragmenten, die spezifisch sind für Endothelzellen, endständig Mannose tragende Glykoproteine, Glykolipide oder Polysaccharide, Zytokine, Wachstumsfaktoren, Adhäsionsmoleküle oder, in einer besonders bevorzugten Ausführungsform, aus Glykoproteinen aus der Hülle von Viren, die einen Tropismus für Endothelzellen haben.

In einer anderen bevorzugten Ausführungsform kann der Ligand spezifisch an glatte Muskelzellen binden und ist ausgewählt aus der Gruppe umfassend monoklonale Antikörper oder deren Fragmente, die spezifisch sind für Aktin, Zellmembranrezeptoren sowie Wachstumsfaktoren oder, in einer besonders bevorzugten Ausführungsform, aus Glykoproteinen aus der Hülle von Viren, die einen Tropismus für glatte Muskelzellen haben.

In einer weiteren bevorzugten Ausführungsform kann der Ligand (b) spezifisch binden an Makrophagen und/oder Lymphozyten und ist ausgewählt aus der Gruppe umfassend monoklonale Antikörper, die spezifisch sind für Membranantigene auf Makrophagen und/oder Lymphozyten, intakte Immunglobuline oder Fc-Fragmente von polyklonalen oder monoklonalen Antikörpern, die spezifisch sind für Membranantigene auf Makrophagen und/oder Lymphozyten, Zytokine, Wachstumsfaktoren, Mannose-endständig tragende Peptide, Proteine, Lipide oder Polysaccharide oder, in einer besonders bevorzugten Ausführungsform, aus Glykoproteinen aus der Hülle von Viren, insbesondere das HEF-Protein vom Influenza C-Virus mit Mutation in der Nukleotidposition 872 oder HEF-Spaltprodukte des Influenza C-Virus enthaltend die katalytische Triade Serin-71, Histidin 368 oder -369 und Asparaginsäure 261.

Bei einer weiteren bevorzugten Ausführungsform kann der Ligand (b) spezifisch binden an Gliazellen und ist ausgewählt aus der Gruppe umfassend Antikörper und Antikörperfragmente, die spezifisch binden an Membranstrukturen von Gliazellen, Adhäsionsmoleküle, endständig Mannose tragende Peptide, Proteine, Lipide oder Polysaccharide, Wachstumsfaktoren oder, in einer besonders bevorzugten Ausführungsform, aus Glykoproteinen aus der Hülle von Viren, die einen Tropismus für Gliazellen haben.

Eine weitere bevorzugte Ausführungsform zeichnet sich dadurch aus, daß der Ligand (b) spezifisch binden kann an blutbildende Zellen und ausgewählt ist aus der Gruppe umfassend Antikörper oder Antikörperfragmente, die spezifisch sind für einen Rezeptor des Stern cell factors, IL-1 (insbesondere Rezeptortyp I oder II), IL-3 (insbesondere Rezeptortyp a oder β), IL-6 oder GM-CSF, sowie intakte Immunglobuline oder Fc-Fragmente, die diese Spezifität aufweisen und Wachstumsfaktoren wie SCF, IL-1, IL-3, IL-6 oder GM-CSF sowie deren Fragmente, die an die zugehörigen Rezeptoren binden.

Bei einer anderen bevorzugten Ausführungsform kann der Ligand (b) spezifisch binden an Leukämiezellen und ist ausgewählt aus der Gruppe umfassend Antikörper, Antikörperfragmente, Immunglobuline oder Fc-Fragmente, die spezifisch binden an Membranstrukturen auf Leukämiezellen, wie CD13, CD14, CD15, CD33, CAMAL, Sialosyl-Le, CD5, CD1e, CD23, M38, IL-2-Rezeptoren, T-Zell-Rezeptoren, CALLA oder CD19, sowie Wachstumsfaktoren oder davon abstammende Fragmente oder Retinoide.

Eine andere bevorzugte Ausführungsform zeichnet sich dadurch aus, daß der Ligand (b) spezifisch an virusinfizierte Zellen binden kann und ausgewählt ist aus der Gruppe umfassend Antikörper, Antikörperfragmente, intakte Immunglobuline oder Fc-Fragmente, die spezifisch sind für ein Virusantigen, das nach Infektion durch das Virus auf der Zellmembran der infizierten Zelle exprimiert wird.

Schließlich kann es sich bei dem Liganden (b) auch um einen solchen handeln, der spezifisch binden kann an Bronchialepithelzellen, sinusoidale Zellen der Leber oder Leberzellen und der bevorzugt ausgewählt ist aus der Gruppe umfassend Transferrin, Asialoglycoproteine, wie Asialoorosomucoid, Neoglycoprotein oder Galactose, Insulin, endständig Mannose tragende Peptide, Proteine, Lipide oder Polysaccharide, intakte Immunglobuline oder Fc-Fragmente, die spezifisch an die Zielzellen binden und, in einer besonders bevorzugten Ausführungsform, aus Glykoproteinen aus der Hülle von Viren, die spezifisch an die Zielzellen binden.

In bevorzugter Ausführungsform ist das Fusionsprotein (c) ausgewählt aus dem Haemagglutinin von Influenza A- oder B-Viren, der HA2-Komponente des Haemagglutinins von Influenza A- oder B-Viren sowie Peptidanaloga hiervon, das M2-Protein von Influenza A-Viren, das HEF-Protein von Influenza C-Viren, Transmembranproteinen von Filoviren, wie Marburg-Virus oder Ebola-Virus, Transmembranglykoproteine von Tollwutvirus, Vesicular Stomatitis Virus, Semliki Forest-Virus, Tickborn Encephalitis-Virus, Fusionsproteinen des HIV-Virus, Sendai-Virus (insbesondere die F1-Komponente) oder des respiratorischen syncytialen Virus (insbesondere die gp37-Komponente) sowie Fragmente dieser viralen Fusionsproteine oder der Transmembranglykoproteine, welche die fusiogenen Peptide enthalten.

In bevorzugter Ausführungsform liegt das einzuführende Gen (d) in Form eines Plasmids vor.

Die erfindungsgemäßen Vektoren können als Arzneimittel oder Bestandteil eines Arzneimittels eingesetzt werden, wobei die Vektoren bevorzugt zur Herstellung eines Arzneimittels für die intravenöse, intraarterielle, intraportale, intrakraniale, intrapleurale, intraperitoneale oder lokale Einführung eines gewünschten Gens in bestimmte Zielzellen verwendet werden.

Durch die erfindungsgemäßen zielzellspezifischen Vektoren wird erreicht, daß nach parenteraler, bevorzugt intravenöser oder intraarterieller Verabreichung des Vektors dessen Konzentration an der Zielzelle erhöht und damit die Transfektionsrate der Zielzelle gesteigert werden kann. Dieser erfindungsgemäße Vorteil wird erreicht durch eine synergistische Wirkung der einzelnen miteinander verbundenen Komponenten der erfindungsgemäßen Vektoren.

Die zielzellspezifischen Liganden weisen eine hohe Spezifität für die gewünschten Zielzellen auf. In bevorzugter Ausführungsform handelt es sich hierbei um Virushüllproteine, die für bestimmte Zellen spezifisch sind. Je nach der gewünschten Zielzelle wird ein geeignetes Virushüllprotein ausgewählt. Da diese zielzellspezifischen Liganden, insbesondere die Virushüllproteine, an den Träger für das gewünschte Gen gekoppelt werden, wird erreicht, daß das gewünschte Gen über den zielzellspezifischen Liganden an die Zielzelle bindet und sich an der Zielzelle anreichert.

Die nicht-viralen Träger für das Gen sind in bevorzugter Weise solche Verbindungen, die bekanntermaßen eine lange Verweilzeit im Blut haben. Durch diese längere Verweilzeit im Blut wird eine möglichst lange Exposition der Zielzelle mit einer möglichst hohen Konzentration des Vektors erzielt, um hierdurch eine maximal mögliche Bindung von Vektoren an die Zielzellen über die zielzellspezifischen Liganden zu erreichen. In besonders bevorzugter Ausführungsform werden diese nicht-viralen Träger kationisiert, damit eine Komplexierung mit der negativ geladenen DNA ermöglicht wird.

Weiterhin weisen die erfindungsgemäßen Vektoren Fusionsproteine auf, die für die Penetration des Vektors aus den Endosomen oder Lysosomen in das Zytoplasma der Wirtszelle verantwortlich sind. Unter Fusionsproteinen im Sinn der vorliegenden Erfindung werden also solche Proteine verstanden, die einen Eintritt des Vektors in das Zytoplasma der Zielzelle ermöglichen. Derartige Fusionsproteine sind vor allem von Viren bekannt.

Das einzuführende Gen kann in Form von Nukleinsäure kodierend für das entsprechende Gen vorliegen, das gegebenenfalls mit den entsprechenden Steuerbereichen wie Promotoren etc. versehen ist. In bevorzugter Ausführungsform liegt das einzuführende Gen in Form eines Plasmids vor.

Die erfindungsgemäß einsetzbaren nicht-viralen Träger für das Gen sind an sich bekannt. Nicht-virale Träger wurden übersichtlich beschrieben von Cotten et al., Curr. Biol. 4, 705 (1993); Behr, Acc. Chem. Res. 26, 274 (1993); Felgner, Adv. Deliv. Rev. 5, 163 (1990); Behr, Bioconju▼gate Chem. 5, 382 (1994); Ledley, Hum. Gene Ther. 6, 1129 (1995). Bevorzugt eingesetzt werden im Rahmen der vorliegenden Erfindung Liposomen, kationische Liposomen, die hergestellt werden unter Verwendung von kationischen Lipiden wie Stearylaminen, im Gemisch mit neutralen Phospholipiden, Dioctadecyldimethylammoniumbromid (DDA), im Gemisch mit neutralen Phospholipiden, N[1(2,3,-dioleyloxy)propyl]-N,N,N-trimethylammoniumbromid (DOTMA), 3β[N-(N',N'-dimethylamino-ethan)-carbamoyl]-cholesterol (DC-Chol), 1,2,-Dimyristyloxy-propyl-3-dimethyl-hydroxyethylammoniumbromid (DMRIE) Dime-thyldioctadecylammoniumbromid (DDAB), 1,2,-Dioleoyloxy-3-(trimethylammonio)propan (DOTAP).

Als nicht-virale Träger sind auch kationische Polypeptide und Proteine geeignet wie beispielsweise Polylysin, Protaminsulfate, Histone, Polyornithin, Polyarginin oder kationische amphiphile Lipopolyamine wie beispielsweise Dioctadecylamidoglycylspermin (DOGS), Dipalmitoylphosphati-dylethanolamidospermin (DPPES), N-t-Butyl-N'-tetradecyl-3-tretradecylaminopropionamid (diC14-amidin), DoTB, ChoTB, DoSC, ChoSC, LPLL, DEBDA, DTAB, TTAB, CTAB oder TMAG oder ♂ kationische Polysaccharide, wie beispielsweise Diethylaminoethyldextran sowie kationische organische Polymere wie beispielsweise Polybrene.

In einer weiteren bevorzugten Ausführungsform können zur Erhöhung der Transfektionsrate Formulierungen von kationischen Lipiden und Lipopolyaminen (komplexiert mit DNA) ergänzt sein durch Zumischung von neutralen Phospholipiden, wie Dioleoylphosphatidylethanolamine (DOPE).

In besonders bevorzugter Ausführungsform handelt es sich bei den nicht-viralen Trägern um Verbindungen, deren Grundsubstanzen kationische oder kationisierte wasserlösliche Polypeptide, Proteine, Glykoproteine, Lipoproteine oder Polysaccharide sind, die durch Einführung (gegebenenfalls zusätzlicher) lipophiler Gruppen ein amphiphiles Verhalten aufweisen. Bei den Grundsubstanzen handelt es sich bevorzugt um wasserlösliche Träger, wie Proteine, Glykoproteine, Lipoproteine oder Polysaccharide. In besonders bevorzugter Ausführungsform handelt es sich bei dem Träger um Albumin oder Xylan.

Als kationische Gruppen sind solche Struktureinheiten mit positiver Ladung geeignet, die an die Grundsubstanz gebunden werden können. Bevorzugt handelt es sich bei den kationischen Gruppen um Struktureinheiten, die unter physiologischen Bedingungen Amino-, Guanidino- oder Imidazolyl-Funktionen aufweisen. Die kationischen Gruppen können an die Grundsubstanzen mit den bekannten Methoden der Konjugation gekoppelt werden. Für Aminofunktionen kommt beispielsweise die Kopplung mit Diaminen wie Ethylendiamin oder Hexamethylendiamin in Frage. Freie Aminogruppen können auch mit Methyliodid methyliert werden oder es kann eine Reaktion einseitig abreagierter Glutaraldehydgruppen mit Girard T Reagenz erfolgen [Roser, Dissertation Basel (1990)].

Die Einführung von Guanidino- und Imidazolyl-Gruppen erfolgt durch Kopplung der entsprechenden basischen Aminosäuren mit den nachfolgend beschriebenen Methoden. Bevorzugt wird eine Kopplung an Albumin mit Hilfe von Glutaraldehyd (Roser, Dissertation Basel (1990)).

Die Anzahl der einzuführenden kationischen Gruppen hängt ab von der Anzahl der anionischen Ladung des Genes bzw. der Nukleotidsequenz, mit welcher der Träger komplexiert werden soll. Vorzugsweise sollte der Komplex insgesamt eine neutrale oder kationische Ladung aufweisen.

Als lipophile Gruppen gelten alle Struktureinheiten, die zu einer Erhöhung der Löslichkeit in organischen Lösungsmitteln, wie beispielsweise Octanol, führen.

Als lipophile Gruppen gelten insbesondere ungesättigte Fettsäuren, wie z.B. Ölsäure, die als Ester, Säurechloride und Säureanhydride Anwendung finden.

Die Einführung der lipophilen Gruppen erfolgt mit bekannten Methoden der Konjugation, wie z.B. durch Acylierung, d.h. Umsetzung von Säurechloriden, Säureanhydriden und Estern mit primären und sekundären Aminen, wie bei Seebach, Angew. Chemie 81, 690 (1969) und Satchell, Quart. Rev. 17, 160 (1963) beschrieben.

Die Anzahl der einzuführenden lipophilen Gruppen hängt ab von dem Grad der Lipophilie der Grundsubstanz.

Als Liganden, die spezifisch an die gewünschte Zielzelle binden können, kann eine Vielzahl von Strukturen Verwendung finden. Die Auswahl der geeigneten Liganden richtet sich nach den Zielzellen, für die der Vektor spezifisch sein soll. Bei den Liganden handelt es sich in der Regel um Proteine, Polypeptide oder Glykoproteine, welche eine hohe spezifische Affinität zu Membranbestandteilen auf ausgewählten Zellen (Zielzelle) aufweisen. In Abhängigkeit von der Zielzelle können im Rahmen der vorliegenden Erfindung die folgenden Liganden Verwendung finden:

### 1) Liganden für Endothelzellen

### a) Nicht-virale Liganden

Als Liganden dienen Substanzen, welche bevorzugt an die Oberfläche von Endothelzellen, insbesondere von proliferierenden Endothelzellen, binden. Hierzu gehören Antikörper oder Antikörperfragmente, gerichtet gegen Membranstrukturen von Endothelzellen, wie sie beispielsweise von Burrows et al. (Pharmac. Ther. 64, 155 (1994)); Hughes et al. (Cancer Res. 49, 6214 (1989)) und Maruyama et al., (PNAS-USA 87, 5744 (1990)) beschrieben wurden. Insbesondere zählen hierzu Antikörper gegen die VEGF-Rezeptoren.

Die murinen monoklonalen Antikörper sind bevorzugt in humanisierter Form einzusetzen. Die Humanisierung erfolgt in der von Winter et al. (Nature 349, 293 (1991)) und Hoogenbooms et al. (Rev. Tr. Transfus. Hemobiol. 36, 19 (1993)) dargestellten Weise. Antikörperfragmente werden entsprechend dem Stand der Technik hergestellt, beispielsweise in der von Winter et al., Nature 349, 293 (1991); Hoggenbooms et al., Rev. Tr. Transfus. Hemobiol. 36, 19 (1993); Girol, Mol. Immunol. 28, 1379 (1991) oder Huston et al., Intern. Rev. Immunol. 10, 195 (1993) beschriebenen Weise.

Zu den Liganden gehören desweiteren alle Wirkstoffe, welche an Membranstrukturen oder Membranrezeptoren auf Endothelzellen binden. Beispielsweise gehören hierzu Kinine und Analoge und Homologe von Kininen, die an Kininrezeptoren binden, desweiteren Substanzen, die endständig Mannose enthalten des weiteren IL-1 oder Wachstumsfaktoren oder deren Fragmente bzw. Teilsequenzen von ihnen, die an Rezeptoren exprimiert durch Endothelzellen binden, wie beispielsweise PDGF, bFGF, VEGF, TGFβ (Pusztain et al., J. Pathol. 169, 191 (1993)). Des weiteren gehören hierzu Adhäsionsmoleküle, welche an aktivierte und/oder proliferierende Endothelzellen binden. Derartige Adhäsionsmoleküle, wie beispielsweise SLex, LFA-1, MAC-1, LECAM-1 oder VLA-4, wurden bereits beschrieben (Übersichten bei Augustin-Voss et al., J. Cell. Biol. 119, 483 (1992); Pauli et al., Cancer Metast. Rev. 9, 175 (1990); Honn et al., Cancer Metast. Rev. 11, 353 (1992)).

### b) Virale Liganden

Zu den Liganden im Sinne dieser Erfindung gehören insbesondere Glykoproteine der Hülle von Viren, die einen Tropismus für Endothelzellen haben. Zu diesen Viren gehören beispielsweise:
- Filoviren, beispielsweise
   - das Marburg-Virus
      mit seinem Hüllprotein GP (glycoprotein) und sGP (second glycoprotein)
      (Kiley et al., J. General Virology 69, 1957 (1988); Will et al., J. Virol. 67, 1203 (1993); Schnittler et al., J. Clin. Invest. 91, 1301 (1993); Feldmann et al., Virus Res. 24, 1 (1992))
   - oder das Ebola-Virus
      jeweils mit seinem Hüllprotein GP und sGP
      (Schnittler et al., J. Clin. Invest. 91, 1301 (1993); Volchov et al., Virol. 214, 421 (1995); Jahrling et al., Lancet 335, 502 (1990), Feldmann et al., Arch. Virol. 7, 81 (1993); Geisbert et al., J. Comp. Path. 106, 137 (1992))
- das Cytomegalovirus,
   besonders mit seinem gB-Protein
   (Waldman et al., Transplant. Proc. 27, 1269 (1995); Sedmak et al., Transplant. 58, 1379 (1994); Sedmak et al., Archives Virol. 140, 111 (1995); Koskines, Transplant. 56, 1103 (1993); Scholz et al., Hum. Immunol. 35, 230 (1992), Alcami et al., J. Gen. Virol. 72, 2765 (1991); Poland et al., J. Infect. Dis. 162, 1252 (1990); Ho et al., J. Infect. Dis. 150, 956 (1984); Spaete et al., J. Virol. 64, 2922 (1990))
- das Herpes simplex-Virus Type I
   (Etingin et al., PNAS 90, 5153 (1993); Key et al., Lab. Invest. 68, 645 (1993); Kubota et al., J. Immunol. 138, 1137 (1987))
- das HIV-1 Virus
   (Scheylovitova et al., Arch. Virol. 140, 951 (1995); Lafon et al., AIDS 8, 747 (1994); Re et al., Microbiologica 14, 149 (1991))
- das Masern-Virus
   (Mazure et al., J. Gen. Virol. 75, 2863 (1994))
- das Hantaan-Virus
   (Pensiero et al., J. Virol. 66, 5929 (1992); Zhu, Chinese Med. J. 68, 524 (1988))
- Alphaviren, wie Semliki Forest-Virus
   (Jakob, J. Med. Microbiol. 39, 26 (1993))
- das Virus des epidemischen, haemorrhagischen Fiebers
   (Yi, Chinese J. Pathol. 21, 177 (1992))
- das Poliovirus
   (Condere et al., Virol. 174, 95 (1990))
- Enteroviren (wie z.B. Echo 9, Echo 12, Cochsackie B3)
   (Kirkpatrick et al., Am. J. Pathol. 118, 15 (1985)).

### 2) Liganden für glatte Muskelzellen

### a) Nicht-virale Liganden

Als Liganden sind beispielsweise Antikörper oder Antikörperfragmente, gerichtet gegen Membranstrukturen von glatten Muskelzellen zu verwenden, Hierzu gehört
- der Antikörper 10F3 (Printseva et al., Exp. Cell Res. 169, 85 (1987); American J. Path. 134, 305 (1989))
- Antikörper gegen Actin
- Antikörper gegen Angiotensin II-Rezeptoren
- Antikörper gegen Rezeptoren für Wachstumsfaktoren oder Antikörper gerichtet beispielsweise gegen
- EGF-Rezeptoren
- PDGF-Rezeptoren
- FGF-Rezeptoren
- gegen Endothelin A-Rezeptoren.

Zu den Liganden gehören des weiteren alle Wirksubstanzen, welche an Membranstrukturen oder Membranrezeptoren auf glatten Muskelzellen binden (Übersicht bei Pusztai et al., J. Pathol. 169, 191 (1993), Harris, Current Opin. Biotechnol. 2, 260 (1991)). Beispielsweise gehören hierzu Wachstumsfaktoren oder deren Fragmente bzw. Teilsequenzen von ihnen, die an Rezeptoren exprimiert durch glatte Muskelzellen binden wie beispielsweise
- PDGF
- EGF
- TGFβ
- TGFa
- FGF
- Endothelin A

### b) Virale Liganden

Zu den Liganden im Sinne dieser Erfindung gehören jedoch besonders Glykoproteine der Hülle von solchen Viren, die einen Tropismus für glatte Muskelzellen haben. Zu diesen Viren gehört beispielsweise das Cytomegalovirus (Speir et al., Science 265, 391 (1994)).

### 3) Auswahl des Liganden für Makrophagen und Lymphozyten

### a) Nicht-virale Liganden

Zu den Liganden, die an die Oberfläche von Makrophagen und Lymphozyten spezifisch binden, gehören Antikörper oder Antikörperfragmente gerichtet gegen Membranstrukturen von Immunzellen, wie sie beispielsweise von Powelson et al., Biotech. Adv. 11, 725 (1993) beschrieben wurden.

Des weiteren gehören zu den Liganden auch monoklonale oder polyklonale Antikörper oder Antikörperfragmente, die mit ihren konstanten Domänen an Fc-g- oder Fc-e-Rezeptoren von Immunzellen binden (Rojanasakul et al., Pharm. Res. 11, 1731 (1994)), insbesondere gehört hierzu das Fc-Fragment von humanem polyklonalem Immunglobulin. Derartige Fc-Fragmente werden beispielsweise entsprechend der Methoden von Haupt et al., Klin. Wschr. 47, 270 (1969); Kranz et al., Dev. Biol. Standard 44, 19 (1979); Fehr et al., Adv. Clin. Pharmac. 6, 64 (1974); Menninger et al., Immunochem. 13, 633 (1976) hergestellt.

Zu den Liganden gehören des weiteren alle Substanzen, welche an Membranstrukturen oder Membranrezeptoren auf der Oberfläche von Immunzellen binden. Beispielsweise gehören hierzu die Zytokine IL-1, IL-2, IL-3, IL-4, IL-6, IL-10, TNFa, GM-CSF, M-CSF des weiteren Wachstumsfaktoren wie EGF, TGF, FGF, IGF oder PDGF, oder deren Fragmente bzw. Teilsequenzen von ihnen, die an Rezeptoren exprimiert durch derartige Zellen binden.

Hierzu gehören des weiteren Liganden, welche an Zellmembranstrukturen, wie beispielsweise den Mannose 6-Phosphat-Rezeptor auf Makrophagen in Milz, Leber, Lunge und andere Gewebe binden. Diese Liganden und Membranstrukturen sind übersichtlich bei Perales et al., Eur. J. Biochem. 226, 255 (1994) beschrieben.

### b) Virale Liganden

Zu den Liganden im Sinne dieser Erfindung gehören jedoch besonders Glykoproteine der Hülle von solchen Viren, die einen Tropismus für Lymphozyten und/oder Makrophagen haben.

Zu diesen Makrophagen infizierenden Viren gehören beispielsweise:
- HIV-1,
   besonders solche Stämme mit Mutationen in der V3-Region von gp120, die zu einer erhöhten Bindung an Makrophagen führen, z.B. beschrieben von Kim et al., J. Virol. 69, 1755 (1995); Valentin et al., J. Virol. 68, 6684 (1994); Collin et al., J. Gen. Virol. 75, 1597 (1994); Shoida et al., PNAS 89, 9434 (1992); Chesebro et al., J. Virol. 66, 6547, (1992), Shaw et al., J. Virol. 66, 2577 (1992); Liu et al., J. Virol. 64, 6148 (1990); Broder et al., PNAS 92, 9004 (1995); Cangue et al., Virol. 208, 779 (1995)
- HIV-2
   (Valentin et al., J. Virol. 68, 6684 (1994))
- Hantaviren, beispielsweise das Punmalavirus
   (Temonen et al., Virol. 206, 8 (1995)
- Cytomegalovirus
   (Fajac et al., Am. J. Resp. Crit. Care Med. 149, 495 (1994); Kondo et al., PNAS 91, 11879 (1994); Ibanez et al., J. Virol. 65, 6581 (1991))
- Respiratory Syncytial Virus
   (Becker et al., Am. J. Resp. Cell Mol. Biol. 6, 369 (1992); Roberts, Infect. Immun. 35, 1142 (1982))
- Herpes simplex-Virus
   (Plaeger-Marshall et al., Pediatric Res. 26, 135 (1989))
- Filoviren
   (Schnittler et al., J. Clin. Invest. 91, 1301 (1993); Zaki, Eur. Conf. Tropical Med. p2 (A22), Hamburg, Germany (1995).

Zu den Lymphozyten infizierenden Viren gehören beispielsweise:
- Varizella-Zoster-Virus (VZV)
   VZV infiziert besonders T-Zellen
   (Moffat et al., J. Virol. 69, 5236 (1995))
- Herpes Virus 6 (HHV-6).
   HHV-6 infiziert besonders T-Zellen
   (Takahashi et al., J. Virol. 63, 3161 (1989); Lusso et al., J. Exp. Med. 181, 1303 (1995); Frenkel et al., Adv. Exp. Med. Biol. 278, 1 (1990))
- Rabies-Virus
   Rabies Virus Hüllprotein bindet besonders an TH2-Zellen (Martinez-Arends et al., Clin. Immunol. Immunopath. 77, 177 (1995))
- HIV-1
   Das Glykoprotein gp120 bindet bevorzugt an das CD4 Molekül von T-Zellen
   (Heinkelein et al., J. Virol. 69, 6925 (1995))
- HTLV-II
   HTLV-II infiziert besonders B-Zellen
   (Casoli et al., Virol. 206, 1126 (1995))
- HTLV-I
   HTLV-I infiziert besonders T-Zellen
   (Persaud et al., J. Virol. 69, 6297 (1995); Boyer et al., Cell Immunol. 129, 341 (1990))
- Influenza C-Viren
   Influenza C-Viren binden über das Haemagglutinin-Esterase-Fusions-(HEF)-Protein an N-acetyl-9-β-acetylneuraminsäure (Neu 5,9 Ac), welche bevorzugt auf B-Lymphozyten, weniger oder nicht auf T-Lymphozyten vorkommt
   (Herrler et al., EMBO-J. 4, 1503 (1985); Kamerling et al., BBA 714, 351 (1982); Rogers et al., J. Biol. Chem. 261, 5947 (1986))
- Influenza C-Viren mit Mutation in der Nukleotidposition 872
   (die die Position 284 der Aminosäuresequenz des HEF kodiert),
   beispielsweise ein Austausch des Threonins durch Isoleucin.
   Das Oberflächenprotein HEF mit dieser Mutation hat eine deutlich stärkere Affinität zum N-acetyl-9-0-acetylneuraminsäure-Rezeptor als das Wildvirus.
   (Szepanski et al., Virol. 188, 85 (1992))
- HEF Spaltprodukte des Influenza C-Virus, welche die Bindestruktur für N-acetyl-9-β-acetylneuraminsäure enthalten.
   Diese Bindestruktur ist definiert durch die katalytische Triade Serin-71, Histidin 368 oder - 369 und Arsparaginsäure 261
   (Pleschka et al., J. Gen. Virol. 76, 2529 (1995))
- Epstein-Barr Virus.
   EBV infiziert besonders B-Zellen
   (Miller-Yale, J. Biol. Med. 55, 305 (1982); Garzelli et al., Immunol. Lett. 39, 277 (1994); Counter et al., J. Virol. 68, 3410 (1994); Wang et al., J. Virol. 62, 4173 (1988))
- Herpes simplex-Virus-2.
   HSV-2 infiziert besonders an T-Zellen (Kucera et al., Viral Immun. 2, 11 (1989))
- Masernvirus
   (Jacobson et al., J. Gen. Virol. 63, 351 (1982))

### 4) Auswahl der Liganden für Gliazellen

### a) Nicht-virale Liganden

Als Ligand sind des weiteren Substanzen anzusehen, welche an die Oberfläche von Gliazellen binden. Hierzu gehören Antikörper oder Antikörperfragmente gerichtet gegen Membranstrukturen von Gliazellen, wie sie beispielsweise von Mirsky et al. (Cell and Tissue Res. 240, 723 (1985)); von Coakham et al., (Prog. Exp. Tumor Res. 29, 57 (1985)) und von McKeever et al. (Neurobiol. 6, 119 (1991)) berichtet wurden. Zu diesen Membranstrukturen gehören des weiteren Neuraladhäsionsmoleküle wie N-CAM, insbesondere dessen Polypeptidkette C (Nybroe et al., J. Cell Biol. 101, 2310 (1985)).

Hierzu gehören des weiteren alle Wirkstoffe, welche an Membranstrukturen oder Membranrezeptoren auf Gliazellen binden. Beispielsweise gehören hierzu Substanzen, die endständig Mannose tragen und an den Mannose-6-Phosphatrezeptor binden (Perales et al., Eur. J. Biochem. 226, 225 (1994), Insulin und Insulin-like growth factor (Merrill et al., J. Clin. Endocrin. Metab. 71, 199 (1990)), PDGF (Ek et al., Nature 295, 419 (1982), und diejenigen Fragmente dieser Wachstumsfaktoren, welche an die zugehörigen Membranrezeptoren binden.

### b) Virale Liganden

Zu den Liganden im Sinne der Erfindung gehören insbesondere Glykoproteine der Hülle von solchen Viren, die einen Tropismus für Gliazellen haben.

Zu diesen Viren gehören beispielsweise:
- HIV-1 Subtyp JRF1
   (Sharpless et al., J. Virol. 66, 2588 (1992)
- Herpes simplex-virus I
   (Xie et al., Eye Science (Yen Ko Hsueh Pao) 10, 67 (1994); Genis et al., J. Exp. Med. 176, 1703 (1992))

### 5) Nicht-virale Liganden für blutbildende Zellen

Zu den Liganden gehören Antikörper oder Antikörperfragmente, die gerichtet sind gegen Rezeptoren, die insbesondere exprimiert werden auf gering differenzierten Blutzellen.

Derartige Antikörper sind beispielsweise für folgende Rezeptoren beschrieben worden:
- Stern Cell Factor Receptor
- IL-1-Rezeptor (Type I)
- IL-1-Rezeptor (Type II)
- IL-3-Rezeptor a
- IL-3-Rezeptor β
- IL-6-Rezeptor
- GM-CSF-Rezeptor.

Des weiteren gehören zu den Liganden auch monoklonale oder polyklonale Antikörper oder Antikörperfragmente, die mit ihren konstanten Domänen an Fc-g Rezeptoren von Immunzellen binden (Rojanasakul et al., Pharm. Res. 11, 1731 (1994)).

Zu den Liganden gehören des weiteren Substanzen, welche an Membranstrukturen oder Membranrezeptoren auf der Oberfläche von gering differenzierten Blutzellen binden. Beispielsweise gehören hierzu Wachstumsfaktoren, wie SCF, IL-1, IL-3, IL-6, GM-CSF oder deren Fragmente bzw. Teilsequenzen von ihnen, die an Rezeptoren exprimiert durch derartige Zellen binden.

### 6) Nicht-virale Liganden für Leukämiezellen und Tumorzellen

Zu den Liganden, welche an die Oberfläche von Leukämiezellen binden, gehören Antikörper oder Antikörperfragmente, gerichtet gegen Membranstrukturen von Leukämiezellen. Eine große Anzahl derartiger monoklonaler Antikörper sind bereits für diagnostische und therapeutische Verfahren beschrieben worden (Übersichten bei Kristensen, Danish Medical Bulletin 41, 52 (1994); Schranz, Therapia Hungarica 38, 3 (1990); Drexler et al., Leuk. Rex. 10, 279 (1986); Naeim, Dis. Markers 7, 1 (1989); Stickney et al., Current Op. Oncol. 4, 847 (1992); Drexler et al., Blut 57, 327 (1988); Freedment et al., Cancer Invest. 9, 69 (1991)). Je nach Typ der Leukämie sind als Liganden beispielsweise folgende monoklonale Antikörper oder deren antigenbindende Antikörperfragmente geeignet:

| Zellen | Membranantigen | monoklonale Antikörper beschrieben von |
|---|---|---|
| AML | CD13 | Kaneko et al., Leuk. Lymph. 14, 219 (1994) |
| | - | Muroi et al., Blood 79, 713 (1992) |
| | CD14 | Ball, Bone Marrow Transplnt. 3, 387 (1988) |
| | CD15 | Guyotat et al., Bone Marrow Transplant. 6, 385 (1990) |
| | CD33 | Jurcic et al., Leukemia 9, 244 (1995) |
| | | Caron et al., Cancer 73, 1049 (1994) |
| | CAMAL | Shellard et al., Exp. Hematol. 19, 136 (1991) |
| | Sialosyl-Le | Muroi et al., Blood 79, 713 (1992) |
| B-CLL | CD5 | Kaminski et al., Cancer Treat. Res. 38, 253 (1988) |
| | | Tassone et al., Immunology Lett. 39, 137 (1994) |
| | CD1c | Orazi et al., Eur. J. Haematol. 47, 28 (1991) |
| | CD23 | |
| | Idiotypen und Isotypen der Membranimmunoglobuline Schroeder et al., Immunol. Today 15, 289 (1994) | |
| T-CLL | CD33 | Imai et al., J. Immunol. 151, 6470 (1993) |
| | IL-2-Rezeptoren | Waldmann et al., Blood 82, 1701 (1993) |
| | T-Zell-Rezeptoren | |
| ALL | CALLA | Morishima et al., Bone Marrow Transplant. 11, 255 (1993) |
| | CD19 | Anderson et al., Blood 80, 84 (1993) |
| | Non-Hodgkin | |
| | Lymphoma | Okazaki et al., Blood 81, 84 (1993) |

Zu den nicht-viralen Liganden für Tumorzellen gehören Antikörper und Fragmente dieser Antikörper gerichtet gegen Membranstrukturen auf Tumorzellen. Derartige Antikörper wurden zum Beispiel von Sedlacek et al., Contrib. to Oncol. 32, Karger Verlag, München (1988) und Contrib. to Oncol. 43, Karger Verlag, München (1992) übersichtlich dargestellt.

Weitere Beispiele stellen dar Antikörper gegen:
- Sialyl Lewis
   (Ohta et al., Immunol. Lett. 44, 35 (1995))
- Peptide auf Tumoren, welche von T-Zellen erkannt werden
   (Maeurer et al., Melanoma Res. 6, 11 (1996); Coulie, Stem Cells 13, 393 (1995); Stoh et al., J. Biochem, 119, 385 (1996), Slingluff et al., Curr. Opin. Imunol. 6, 733 (1994))
- von Onkogenen exprimierte Proteine
   (Cheever et al., Immunol. Rev. 145, 33 (1995); Talarico et al., PNAS 87, 4222 (1990))
- Ganglioside wie GD3, GD2, GM2, 9-0-acetyl GD3, Fucosyl GM1
   (Helling et al., Cancer Res. 55, 2783 (1995); Linvingston et al., Vaccine 11, 1199 (1993); Vaccine 12, 1275 (1994); Livingston et al., Cancer Immunol. Immunother. 29, 179 (1989); Gnewuch et al., Int. J. Cancer 8, 125 (1994); Jennemann et al., J. Biochem. 115, 1047 (1994); Ravindranath et al., Cancer Res. 49, 3891 (1989))
- Blutgruppenantigene und deren Vorläufer
   (Springer et al., Cancer 37, 169 (1976); Carbohydrate Res. 179, 271 (1988); Molec. Immunol. 26, 1 (1989); Fung et al., Cancer Res. 50, 4308 (1990))
- Antigene auf dem polymorphen epithelialen Mucin
   (PEM; Burchell et al., Cancer Surreys 18, 135 (1993))
- Antigene auf Heat Shock Proteinen
   (Blackere et al., J. Immunother. 14, 352 (1993)).

Die murinen monoklonalen Antikörper sind bevorzugt in humanisierter Form einzusetzen. Die Humanisierung erfolgt wie bereits beschrieben.
Antikörperfragmente werden, wie bereits beschrieben, entsprechend dem Stand der Technik hergestellt.

Zu den Liganden gehören des weiteren alle Wirkstoffe, welche an Membranstrukturen oder Membranrezeptoren von Leukämiezellen oder Tumorzellen binden. Beispielsweise gehören Steroidhormone oder Peptidhormone hierzu oder auch Wachstumsfaktoren oder deren Fragmente bzw. Teilsequenzen von ihnen, die an Rezeptoren exprimiert durch Leukämiezellen oder Tumorzellen binden.

Derartige Wachstumsfaktoren wurden bereits beschrieben (Übersichten bei Cross et al., Cell 64, 271 (1991); Aulitzky et al., Drugs 48, 667 (1994); Moore, Clin. Cancer Res. 1, 3 (1995); Van Kooten et al., Leuk. Lymph. 12, 27 (1993)). Beispielsweise gehören zu ihnen:
- IFNa bei Non-Hodgkin Lymphomen
- IL-2 besonders bei T-Zell-Leukämien
- FGF bei T-Zell monozytären, -myeloiden, -erythroiden und -megakaryoblastischen Leukämien
- TGFβ bei Leukämien
- Retinoide, z.B. "Retinoic acid" bei akuter promyelozytärer Leukämie.

### 7) Nicht-virale Liganden für infizierte Zellen

Zur Therapie von Infektionserkrankungen gehören zu den Liganden Antikörper oder Antikörperfragmente, gerichtet gegen die Infektionserreger. Beispielsweise sind dies bei Virusinfektionen die Virusantigene exprimiert auf der Zellmembran von virusinfizierten Zellen.

Derartige Antikörper sind beispielsweise für mit folgenden Viren infizierte Zellen beschrieben worden:
- HBV
- HCV
- HSV
- HPV
- HIV
- EBV
- HTLV.

Des weiteren gehören zu den Liganden auch monoklonale oder polyklonale Antikörper oder Antikörperfragmente, die mit ihren konstanten Domänen an Fc-g- oder Fc-e-Rezeptoren von Immunzellen binden.

Die murinen monoklonalen Antikörper sind bevorzugt in humanisierter Form einzusetzen. Die Humanisierung erfolgt wie bereits beschrieben.

Zu den Liganden gehören desweiteren alle Substanzen, welche an Membranstrukturen oder Membranrezeptoren auf der Oberfläche von virusinfizierten Zellen binden. Beispielsweise gehören hierzu Wachstumsfaktoren, wie Zytokine, EGF, TGF, FGF oder PDGF, oder deren Fragmente bzw. Teilsequenzen von ihnen, die an Rezeptoren exprimiert durch derartige Zellen binden.

### 8) Liganden für weitere Parenchymzellen

### a) Nicht-virale Liganden

Hierzu gehören Liganden, welche an Zellmembranstrukturen binden, welche selektiv sind für bestimmte Gewebe. Hierzu zählen beispielsweise:

| Membranstruktur | Liganden | Gewebezellen |
|---|---|---|
| Asialoglycoprotein-Rezeptor | Asialoorosomucoid Neoglycoprotein Galactose | Leberzellen |
| Transferrin-Rezeptor | Transferrin | Leber, andere Gewebezellen |
| Insulin-Rezeptor | Insulin | Makrophagen in Milz, Leber, Lunge, andere Gewebe |
| Fc-γ-Rezeptoren | Immunglobulin G | retikuloendotheliales System, andere Gewebe |

Diese Liganden und Membranstrukturen sind übersichtlich bei Perales et al., Eur. J. Biochem. 226, 255 (1994) beschrieben.

### b) Virale Liganden

Zu diesen Liganden im Sinne der Erfindung gehören jedoch besonders Glykoproteine der Hülle von Viren, die einen Tropismus für ausgewählte Zellen haben, wie beispielsweise für
- Bronchialepithelzellen
   - Respiratory syncytial virus
      (Becker et al., Am. J. Resp. Cell Mol. Biol. 6, 369 (1992))
- Leberzellen
   - Hepatitis C-Virus
      (Uchida et al., Pathol. Internat. 44, 832 (1994); Carloni et al., Arch. Virol. 8, 31 (1993); Prince et al., Curr. Stud. Hemat. Blood Trans. 61, 195 (1994))
   - Filoviren
      Leberzellen binden z.B. das Marburg-Virus über den Asialoglykoprotein-Rezeptor
      (Becker et al., J. Gen. Virol. 76, 393 (1995))
   - Hepatitis B-Virus
      Leberzellen binden bevorzugt über den Asialoglykoprotein-Rezeptor an der preS2 und preS1 Domäne von HBV (Shimizu et al., J. Med. Virol. 20, 313 (1986); Treichel et al., J. Gen. Virol. 75, 3021 (1994); Gerlich et al., J. Hepat. 17/3, 10 (1993); Gripon et al., Virol. 192, 534 (1993); Pontisso et al., Hepatol. 14, 405 (1991); Ochiya et al., PNAS 86, 1875 (1989))
   - Hepatitis D-Virus
      (Colombo et al., J. Hepatol. 12, 64 (1991))
- lebersinusoidale Zellen
   - Hepatitis V-Virus
      HBV wird gebunden über Fibronectin
      (Budhowska et al., J. Virol. 69, 840 (1995)).

### 9) Liganden für die Prophylaxe von Infektionserkrankungen

Für die Prophylaxe von Infektionserkrankungen sind als Liganden alle Substanzen geeignet, welche an Zellmembranstrukturen von Makrophagen und/oder Lymphozyten binden. Derartige Liganden wurden bereits beschrieben.

### Herstellung viraler Liganden

Virale Liganden werden entweder durch Lösung der Envelopeproteine aus einer Virusanreicherung mit Hilfe von Detergentien (wie beispielsweise β-D-octylglucopyranosid) und Abtrennung durch Zentrifugation (Übersicht bei Mannino et al., Bio-Techniques 6, 682 (1988)) gewonnen oder aber mit Hilfe von dem Fachmann bekannter molekularbiologischer Methoden, wie sie in der unter II/2/a-h) zitierten Literatur, beispielsweise für das HEF-Glykoprotein von Pleschka et al. (J. Gen. Virol. 76, 2529 (1995)) und von Szepanski et al. (Virol. 188, 85 (1992)), beschrieben wurden.

### Erfindungsgemäß verwendete Fusionsproteine (c)

Im Rahmen der vorliegenden Erfindung werden Proteine verwendet, welche fusiogene Eigenschaften haben. Derartige Proteine können direkt mit Zellmembranen fusionieren. Eine Reihe von Viren besitzen fusiogene Hüllproteine, so beispielsweise Paramyxoviren, Retroviren und Herpesviren (Gaudin et al., J. Gen. Virol. 76, 1541 (1995)).

Zu den fusiogenen Proteinen im Sinne dieser Erfindung gehören des weiteren auch solche Glykoproteine, welche erst nach Internalisierung in Endosomen und bei sauerem pH mit der Zellmembran oder Endosomen fusionieren.

Eine Reihe von Viren besitzen Glykoproteine, die verantwortlich sind sowohl für die Virusanheftung als auch nachfolgend die Zellmembranfusion (Gaudin et al., J. Gen. Virol. 76, 1541 (1995)).

Derartige Proteine werden beispielsweise von Alpha-, Rhabdo- und Orthomyxoviren gebildet.

### Virale Fusionsproteine

Virale Fusionsproteine im Sinne der Erfindung wurden übersichtlich beschrieben von Hughson, Current Biol. 5, 265 (1995); Hoekstra, J. Bioenergetics Biomembranes 22, 675 (1990); White, Ann. Rev. Physiol. 52, 675 (1990)).

Fusionsproteine im Sinne dieser Erfindung sind beispielsweise:
- das Haemagglutinin von Influenza A- oder B-Viren, insbesondere die HA2-Komponente
   (Stegmann et al., J. Biol. Chem. 266, 18404 (1991); Klenk et al., Virol. 68, 426 (1975); Lazarowitz et al., Virol. 68, 440 (1975); Skehel et al., PNAS 79, 968 (1982); Bosch et al., Virol. 113, 725 (1981))
- das M2-Protein von Influenza A-Viren
   (Sugrue et al., Virol. 180, 617 (1991); Lamb et al., Cell 40, 627 (1985); Pinto et al, Cell 69, 517 (1992); Zebedee et al., J. Virol. 56, 502 (1985); Black et al., J. Gen. Virol. 74, 1673 (1993); Wharton et al., J. Gen. Virol. 75, 945 (1994)) alleine oder in Kombination (Ohuchi et al., J. Virol. 68, 920 (194)) mit dem Haemagglutinin von Influenza eingesetzt oder mit Mutanten von Neuraminidase von Influenza A, denen die Enzymaktivität fehlt, die jedoch Haemagglutination bewirken.
   (Hausmann et al., J. Gen. Virol. 76, 1719 (1995)
- Peptidanaloga des Influenza-Virus Haemagglutinins
   (Wharton et al., J. Gen. Virol. 69, 1847 (1988))
- das HEF-Protein des Influenza C-Virus
   Die Fusionsaktivität des HEF-Proteins wirkt aktiviert durch Spaltung des HEFo in die Untereinheiten HEF1 und HEF2 (Herrler et al., J. Gen. Virol. 69, 839 (1988); Kitane et al., Arch. Virol. 73, 357 (1982); Ohuchi et al., J. Virol. 42, 1076 (1982))
- das Transmembranglykoprotein von Filoviren, wie beispielsweise
   - des Marburg-Virus
      (Feldmann et al., Virol. 182, 353 (1991); Will et al., J. Virol. 67, 1203 (1993); Kiley et al., J. Gen. Virol. 69, 1957 (1988); Geyer et al., Glycobiol. 2, 299 (1992))
   - des Ebola-Virus
      (Elliott et al., Virol. 147, 169 (1985); Cox et al., J. Infect. Dis. 147, 272 (1983); (Kiley et al., J. Gen. Virol. 69, 1957 (1988); Feldmann et al., Arch. Virol. 7, 81 (1993)); Volchkov et al., Virol. 214, 421 (1995)
- das Transmembranglykoprotein des Tollwutvirus
   (Whitt et al., Virol. 185, 681 (1991); Gaudin et al., J. Virol. 65, 4853 (1991); 67, 1365 (1993); Virology 187, 627 (1992))
- das Transmembranglykoprotein (G) des Vesicular Stomatitis-Virus
   (Balch et al., J. Biol. Chem. 261, 14681 (1986); Kreis et al., Cell 46, 929 (1986); Doms et al., J. Cell Biol. 105, 1957 (1987); Zhang et al., J. Virol. 68, 2186 (1994); Ohnishi, Curr. Topics Membr. Transp. 32, 257 (1988); Li et al., J. Virol. 67, 4070 (1993); Zagouras et al., J. Virol. 65, 1976 (1991); Herrmann et al., Biochem. 29, 4054 (1990))
- das Fusionsprotein des HIV-Virus, insbesondere die gp41-Komponente
   (Stareich et al., Cell 45, 637 (1986); Kowalski et al., Science 237, 1351 (1987); Gallaker et al., Cell 50, 327 (1987))
- das Fusionsprotein des Sendai-Virus, insbesondere die F1-Komponente
   (Blumberg et al., J. Gene Virol. 66, 317 (1985); Sechoy et al., J. Biol. Chem. 262, 11519 (1987); Homma und Ohuchi, J. Virol. 12, 1457 (1973); Scheid und Choppin, Virol. 57, 470 (1974))
- das Transmembranglykoprotein des Semliki Forest-Virus, insbesondere die E1-Komponente
   (Omar et al., Virol. 166, 17 (1988); Nieva et al., EMBO-J. 13, 2707 (1994); Phalen et al., J. Cell biol. 112, 615 (1991); Lobigs et al., J. Virol. 64, 1233 + 5214 (1990); Kenney et al., Structure 2, 823 (1994); Garoff et al., Nature 288, 236 (1980); Levy-Mintz et al., J. Virol. 65, 4292 (1991))
- das Transmembranglykoprotein des Tickborn Enzephalitis-Virus
   (Guirakhoo et al., Virol. 169, 90 (1989); J. Gen. Virol. 72, 1323 (1991); Heinz et al., Virol. 198, 109 (1994))
- das Fusionsprotein des menschlichen respiratorischen syncytialen Virus (RSV), insbesondere die gp37-Komponente
   (Collins et al., PNAS 81, 7683 (1984); Elango et al., Nucl. Acids Res. 13, 1559 (1985))

### Herstellung viraler Fusionsproteine

Virale Fusionsproteine werden entweder durch Lösung der Hüllpoteine aus einer Virusanreicherung mit Hilfe von Detergentien (wie beispielsweise β-D-octylglucopyranosid) und Abtrennung durch Zentrifugation (Übersicht bei Mannino et al., Bio▼Techniques 6, 682 (1988)) gewonnen oder aber mit Hilfe von dem Fachmann bekannten molekularbiologischen Methoden. Beispiele für die Herstellung von Fusionsproteinen wurden bereits beschrieben für
- das Influenza-Haemagglutinin
   (Bullough et al., J. Mol. Biol. 236, 1262 (1994); Nature 371, 37 (1994); Daniels et al., Cell 40, 431 (1985); Godley et al., Cell 68, 635 (1992); White et al., Nature 300, 658 (1982); Wiley et al., Ann. Rev. Biochem. 56, 365 (1987); Kawaoka et al., PNAS 85, 321 (1988); Kuroda et al., J. Virol. 63, 1677 (1989), EMBO-J. 5, 1359 (1986); Naeve et al., Virol. 129, 298 (1983); Porter et al., Nature 282, 471 (1972); Hughson, Curr. Biol. 5, 265 (1995))
- das M2-Protein von Influenza V
   (Black et al., J. Gen. Virol. 74, 1673 (1993); Pinto et al., Cell 69, 517 (1992); Zebedee et al., J. Virol. 56, 502 (1985))
- das HEF-Protein von Influenza C
   (Pfeifer et al., Virus. Res. 1, 281 (1984); Herrler et al., Virol. 113, 439 (1981))
- das Transmembranglykoprotein von Filoviren, wie beispielsweise
   - des Marburg-Virus
      (Will et al., J. Virol. 67, 1203 (1993); Feldmann et al., Virus Res. 24, 1 (1992))
   - des Ebola-Virus
      (Volchkow et al., FEBS Lett. 305, 181 (1992); Virol. 214, 421 (1995); Sanchez et al., Virus Res. 29, 215 (1993); Virol. 157, 414 (1987); Eliott et al., Virol. 163, 169 (1985))
- das Transmembranglykoprotein des Tollwutvirus
   (Gaudin et al., J. Virol. 65, 4853 (1991); Virol. 187, 627 (1992); Rose et al., J. Virol. 43, 361 (1982); Witte et al., Virol. 185, 681 (1991))
- das Transmembranglykoprotein des Vesicular Stomatitis-Virus
   (Li et al., J. Virol. 67, 4070 (1993); Riedel et al., EMBO-J. 3, 1477 (1984); Lyles et al., Biochem. 29, 2442 (1990); Metsikko et al., EMBO-J. 5, 3429 (1986))
- das Transmembranglykoprotein des Semliki Forest-Virus
   (Garoff et al., Nature 288, 236 (1980); Kielian et al., J. Virol. 64, 4614 (1990); Kondor-Koch, J. Cell Biol. 97, 644 (1983))
- das Transmembranglykoprotein des Tickborn Enzephalitis-Virus
   (Guirakkoo et al., J. Gen. Virol. 72, 1323 (1991); Heinz et al., Virol. 198, 109 (1994)).

Moleküle mit fusiogener Eigenschaft sind des weiteren:
- Peptide enthaltend die Translokationsdomäne (Domäne II) des Exotoxins A von Pseudomonas (Weis et al., Cancer Res. 52, 6310 (1992)); Fominaga et al., J. Biol. Chem. 271, 10560 (1996))
- Peptide enthaltend das Peptid
   GLFEALLELLESLWELLLEA
   (Gottschalk et al., Gene Ther. 3, 448 (1996))
- Peptide enthaltend das Peptid
   AALAEA[LAEA]₄LAAAAGC (Acm)
   (Wang et al., Technol. Advances in Vector Syst. For Gene Ther., May 6-7, 1996, Coronado, IBC Conference)
- Peptide enthaltend das Peptid
   FAGV-VLAGAALGVAAAAQI
   des Fusionsproteins des Masern-Virus
   (Yeagle et al., Biochem. Biophys. Acta 1065, 49 (1991))
- Peptide enthaltend das Peptid
   GLFGAIAGFIEGGWWGMIDG
   des HA2 Proteins von Influenza A
   (Lüneburg et al., J. Biol. Chem. 270, 27606 (1995))
- Peptide enthalten das Peptid
   GLFGAIAGFIENGWEGMIDGGLFGAIAGFIENGWEGMIDG
   (Burger et al., Biochem 30, 11173 (1991) oder das Peptid
   GLFGAIAGFIE;
   ALFGAIAGFIE;
   LFLGAIAGFIE;
   LLLGAIAGFIE;
   LILGAIAGFIE;
   GIFGAIAGFIE;
   GLLGAIAGFIE;
   GLFAAIAGFIE;
   GLFEAIAGFIE;
   GLFGAMAGFIE;
   GLFGAIAGLIE oder das Peptid
   GLFGAIAGFIV
   (Steinhauer et al., J. Virol 69, 6643 (1995))
   oder das Peptid
   GLFEAIAEFIEGGWEGLIEG
   oder das Peptid
   GLLEALAELLEGGWEGLLEG
   (Ishiguro et al., Biochem. 32, 9792 (1993)).

### Konjugation der Liganden und Fusionsproteine an den Träger

Die Konjugation der Liganden und Fusionsproteine an den Träger wird mit dem Fachmann bekannten Methoden durchgeführt.

### Beispiele für nichtkovalente Bindungen

| | |
|---|---|
| - Träger-Biotin ↔ Avidin-S-S-Ligand | Hashimoto et al., Immunol. 132, 129 (1984) |
| - Träger ↔ bispez. | Raso et al., Immunol. Rev. 62, 93 (1982) |
| Antikörper ↔ Ligand | |

### Beispiele für kovalente Bindungen

| | |
|---|---|
| - Bindung an Protein-NH₂-Gruppen | Carlson et al., Biochem. J. 173, 723 (1978) |
| notwendiges Reagenz: | |

| N-succinimidyl-3-(2-pyrdylthio)-propionat (SPDP) | |
|---|---|
| SDDP-dithiothreitol | Carlson et al., Biochem. J. 173, 723 (1978) |
| 2-iminothiolan | King et al., Biochem. 17, 1499 (1978) |
| 2,2-iminothiolan + 4,4 dithiopyridin | King et al., Biochem. 17, 1499 (1978) |

| 3-methyl-3(4-dithiopyridyl)mercaptopropionimidat | |
|---|---|
| N-acetylhomocysteine thiolacton | Reiner et al., J. Mol. Catal. 2, 335 (1977) |
| acetylmercaptosuccinic anhydrid + NH₂OH | Klotz and Heineg, Arch. Biochem. Biophys. 96, 690 (1979) |
| m-maleimido-benzoyl-N-hydroxysuccinimid ester | Liu et al., Biochem. 18, 690 (1979) |
| succinimidyl-4-(N-maleimidomethylcyclohexan)-1-carboxylat | Yoshitake et al., Eur. J. Biochem. 101, 395 (1979) |
| N-succinimidyliodoacetat | Rector et al., J. Immun. Meth. 24, 321 (1978) |
| 4-hydroxy,3-nitromethylbenzimidat + acetimidat + Na₂S₂O₄ | Müller and Pfleiderer, J. Appl. Biochem. 1, 301 (1979) |
| 4-hydroxy,3-nitromethylbenzimidat + acetimidat + NaNO₂ | Müller and Pfleiderer, J. Appl. Biochem. 1, 301 (1979) |
| oxidiertes Dextran + borohydrid | Hurwith et al., Eur. J. Cancer 14, 1213 (1978) |
| - Bindung an Protein-Hydroxy-Gruppen | |
| notwendiges Reagenz: | |
| cystamin + carbodimid | Erlanger et al., Meth. Imm. Immunochem. 1, 144 (1967) Gilliland, Cancer Res. 40, 3564 (1980) |
| - Bindung an Protein-SH-Gruppen | |
| notwendiges Reagenz: | |
| Protein-SH | Ghose and Blair, CRC Crit. Rev. Ther. Drug Carrier Syst. 3, 263 (1987) |
| Protein-SH + Na₂S₄O₆ | Masuko et al., BBRC 90, 320 (1979) |
| Ellman's reagenz | Raso and Griffin, J. Immunol. 125, 2610 (1980) |
| - Bindung an Protein-Aldehyde-Gruppen | |
| notwendiges Reagenz: | |
| Periodat | Hurwitz et al., Cancer Res. 35, 1175 (1975) |
| - Bindung an Protein-COOH-Gruppen | |
| notwendiges Reagenz: | |
| cystamin + carbodiimid | Gilliland, Cancer Res. 40, 3564 (1980) |

### Auswahl der Nukleotidsequenzen für das einzuführende Gen (d)

Die mit dem Träger zu komplexierenden Nukleotidsequenzen können DNA- oder RNA-Sequenzen sein. Im einfachsten Falle sind es nackte Nukleotidstränge, die das Gen, welches das gewünschte Protein kodiert, beinhalten. Dieses Gen kann ergänzt sein mit zellspezifischen oder virusspezifischen Promotorsequenzen, desweiteren mit Promotormodulen.

Zur Verstärkung und/oder Verlängerung der Expression des Genes können des weiteren virale Promotor- und/oder Enhancersequenzen dem Gen zugefügt werden. Derartige Promotor- und/oder Enhancersequenzen sind beispielsweise von Dillon, TiBTech 11, 167 (1993) übersichtlich dargestellt. Beispielsweise sind derartige Promotor- und/oder Enhancersequenzen
- die LTR-Sequenzen von Rous-Sarcomaviren
- die LTR-Sequenzen von Retroviren
- die Promotor- und Enhancerregion von CMV-Viren
- die ITR-Sequenzen und/oder Promotorsequenzen p5, p19, p40 von AAV-Viren
- die ITR- und/oder Promotorsequenzen von Adenoviren
- die ITR- und/oder Promotorsequenzen von Vaccinia Viren
- die ITR- und/oder Promotorsequenzen von Herpesviren
- die Promotorsequenzen von Parvoviren
- die Promotorsequenzen (upstream regulator region) von Pa-pillomaviren

Vorzugsweise erfolgt der Einbau in ein Plasmid.

### Komplexierung der konjugierten Träger mit dem Gen

Der konjugierte Träger wird mit dem Gen bzw. der Nukleotidsequenz durch Vermischen beider Ausgangssubstanzen komplexiert. Vorzugsweise sollte ein Mischungsverhältnis gewählt werden, welches zu Komplexen mit neutraler oder kationischer Ladung führt.

Bevorzugte Mischungsverhältnisse sind beispielsweise:
- 1 µmol Lipid/20 µg Plasmid
- 1-5 mg Lipid/10-20 µg DNA/RNA
- 6.2 µg Lipid/1.55-3.1 µg DNA
- Lipid/DNA-Peptid (5:1)

Die Beladung erfolgt durch Inkubation des positiv geladenen Trägers mit Genen im gewünschten Mischungsverhältnis. Das Mischungsverhältnis wird (wie von Dittgen et al., Pharmazie 42, 541 (1987) beschrieben) durch Zetapotentialmessung ermittelt.

### Beispiel 1

### Herstellung eines Wirkstoffes zur Transfektion von Endothelzellen

### a) Herstellung des Filovirenglykoproteins als Liganden

Das Filovirusglykoprotein ist ein Hüllprotein mit hoher Affinität zu Endothelzellen. Die Herstellung des Filovirusglykoproteins erfolgt wie von Will et al., J. Virol. 67, 1203 (1993); Feldmann et al., Virus Res. 24, 1 (1992) und Volchkow et al., FEBS Lett. 305, 181 (1992) im Detail beschrieben.

### Herstellung von Ebolaviren

Der Ebolavirus-Subtyp "Zaire" (EBO, Institut für Virologie, Sergiev Posad, Rußland) wurde in Macaca Rhesusaffen passagiert, in Verozellen kultiviert und aus der Zellkulturflüssigkeit isoliert (Volchkow et al., FEBS Lett. 305, 181 (1992)).

### Klonierung und Sequenzierung der viralen RNA

Genomische RNA wurde von gereinigten Viren durch Zentrifugation über Caesiumchloridgradienten isoliert (Volchkow et al. (1992)). Diese RNA diente zur Herstellung einer cDNA-Bibliothek unter Verwendung von Zufallsprimern und einer Reversen Transkriptase vom Myeloblastosisvirus des Huhns. RNA-cDNA Hybride der cDNA-Bibliothek dienten als Ausgangsmaterial für die Amplifikation des GP-Gens mit Hilfe der PCR und folgender synthetischer Primer:
- N1 mit der Sequenz
   5'-GAAGGATCCTGTGGGGCAACAACACAATG (Seq. ID-Nr. 1)
   (komplementär zu den Nukleotiden 114 bis 142 der mRNA, sense) ergänzt mit einer 5'-terminalen BamH1-Region.
- N2 mit der Sequenz
   5'-AAAAAGCTTCTTTCCCTTGTCACTAAA (Seq. ID-Nr. 2)
   (komplementär zu den Nukleotiden 2492 bis 2466 der mRNA, sense) ergänzt mit einer 5'-terminalen Hind-III-Region.

Die Analyse der DNA-Nukleotidsequenz des GP-Gens erfolgte für beide Stränge nach der Methode von Maxam und Gilbert (Methods in Enzymology 65, 499 (1980)). Die Sequenz des EBO GP-Gens Stamm Zaire wurde bei der Genbank hinterlegt unter Nr. U31033 (Volchkow et al. (1992)).

Die Sequenz des EBO GP-Gens war weitgehend identisch zu derjenigen publiziert von Sanchez et al. (Virus res. 29, 215 (1993)). Jedoch wurden in Position 1019 bis 1025 nur sieben anstelle von acht aufeinanderfolgenden A (Adenin; mRNA sense) gefunden.

### Isolierung, Klonierung und Sequenzierung der mRNA spezifisch für das EBO-GP

Mit Hilfe des RNeasy Total RNA Kits (Fa. Quiagen) wurde die mRNA des EBO-GP aus etwa 7 x 107 Verozellen, infiziert mit dem EBO-Virus (1-10 PFU pro Zelle, 1 Tag post infektionem), isoliert.

Für die cDNA Synthese wurden 10 µl der mRNA Lösung (entsprechend etwa 1,4 x 10⁷ infizierte Zellen) mit der Reversen Transkriptase des Myeloblastosisvirus des Huhns in Anwesenheit des Primers
- N3 mit der Sequenz
   oligo-d (T)21, ergänzt mit einer 5'-terminalen Hind-III-Region
inkubiert. Die RNA wurde nachfolgend durch Inkubation des Gemisches mit 1 µg/µl RNAse bei 37° C für 30 min. entfernt.

Die Amplifikation der GP-spezifischen Nukleotidsequenz erfolgte mit Hilfe der PCR unter Benutzung der Primer N1 und N3.

Die PCR wurde durchgeführt im 100 µl Reaktionsansatz enthaltend 1-5 µg cDNA in 50 mM KCl; 10 mM Tris-HCl (pH 8,3), 2 mM MgCl₂; 0,2 mM von jedem Desoxynukleotid und 0,3 µM eines jeden Primers.

Der Reaktionsansatz wurde auf 95° C für 10 min. erhitzt und Taq Polymerase (2,5 U/100 µl) hinzugefügt. 35 Zyklen der DNA Amplifikation wurden durchgeführt. Das Zyklusprogramm beinhaltete 94° C für 1 min.; 70° C für 1 min.; 72° C für 1 min. Nach dem Zyklusprogramm wurden die Proben bei 72° C für die Dauer von 10 min inkubiert. (Alle Komponenten der PCR waren von der Fa. Perkin-Elmer Cetus). Die Produkte der PCR-Reaktion wurden gereinigt (QIA quick Spin PCR Purification Kit; Fa. Quiagen) und die DNA wurde direkt verwendet für die Sequenzierung, für wiederholte PCR-Reaktionen oder für die Klonierung von Plasmidvektoren. Die Nukleotidsequenz der GP-Region, die sich in den ("open reading frames") ORF I und ORF II überlappten, wurden mit Hilfe der Sangertechnik bestimmt (Sanger et al., PNAS 74, 5463 (1977)), wobei folgende Primer Verwendung fanden:
- N4 mit der Sequenz
   5'-CGGACTCTGACCACTGAT (Seq.ID-Nr. 3)
   (komplementär zu den Nukleotiden 1108 bis 1091)
- N5 mit der Sequenz
   5'-TCGTGGCAGAGGGAGTGT (Seq.ID-Nr. 4)
   (komplementär zu den Nukleotiden 1412 bis 1395).

Die PCR-Fragmente wurden in den pGEM2Zf(+)-Vektor kloniert und die rekombinanten Plasmide mit Hilfe der enzymatischen Sequenzierung (Sanger et al., PNAS 74, 5463 (1977)) analysiert.

Die meisten Klone wiesen (ähnlich wie die vRNA) 7 aufeinanderfolgende Adenosine zwischen den Positionen 1018 und 1026 (mRNA sense) auf, während bei etwa 20% der GP-spezifischen mRNA aus EBO infizierten Zellen 8 aufeinanderfolgende Adenosine in diesem Positionsbereich gefunden wurden.

Die mRNA mit 8 Adenosinen kodiert für ein komplettes GP von 676aa (da das 8. Adenosin den "frameshift" von ORF I zu ORF II ermöglicht).

Die mRNA mit nur 7 Adenosinen kodiert demgegenüber ein nichtstrukturelles zweites (second) Glykoprotein (sGP). Entsprechend der Nukleotidsequenz von sGP scheint das sGP identisch zu sein mit dem N-terminalen Teil (274aa) von GP, ergänzt um zusätzlich 70aa, die durch das Ende der ORF I kodiert sind.

Dieses Ende ist im GP nicht vorhanden, da hier das zusätzliche 8. Adenosin die Ablesung von ORF I nach ORF II überführt und ORF I demnach nicht bis zum Ende abgelesen wird.

### Konstruktion von rekombinanten Plasmiden

Die PCR-Produkte, welche die komplette ORF des EBO GP-Gens darstellen, wurden mit den Restriktionsenzymen Bam H I und Hind III inkubiert und ligiert in das Plasmid pGEM3Zf(+), welches vorbehandelt war mit Bam H I und Hind III. Das Plasmid enthält einen T7-Phagen RNA Polyermase Promotor, welcher für die Synthese der EBO GP-RNA mit T7 Polymerase unter Verwendung des Vakzinia Virus/T7 Polymerase Expressionssystems benutzt wurde.

Plasmide, welche die vollständige GP-Nukleotidsequenz der EBO vRNA (7 aufeinanderfolgende Adenosine) enthielten, wurden pGEM-mGP7 und solche die entsprechend die EBO mRNA (8 aufeinanderfolgende Adenosine) enthielten wurden pGEM-mGP8 bezeichnet.

Die GP-spezifischen Nukleotidsequenzen wurden mit Bam H I und Hind III aus den Plasmiden pGEM-mGP7 und pGEM-MGP8 herausgeschnitten, die Enden der entstandenen Fragmente ergänzt mit dem Klenow-Fragment der DNA Polymerase I und verbunden mit der Sma1 Restriktionsstelle des Vektors pSc11 (Fa. Promega, Madison, Wi.) Die entstandenen rekombinanten Plasmide (pSc-mGP7 und pSc-mGP8) wurden für die Herstellung rekombinanter Vakziniaviren benutzt.

### Konstruktion von rekombinanten Vakziniaviren

Rekombinante Vakziniaviren wurden hergestellt mit Hilfe der homologen Rekombination zwischen den tK-Regionen in den rekombinanten Plasmiden (pSC-mGP7 oder pSC-mGP8) und der genomischen DNA des Vakziniavirus (WR-Stamm, wie von Chakrabarti et al. (Mol. Cell. Biol. 5, 3403 (1985)) beschrieben) und mit Hilfe der Lipofectin Transfektionsmethode (Felgner et al., PNAS 84, 7413 (1987)).

Rekombinante Viren wurden gereinigt durch einfache Passagierung auf TK-143-Zellen und vierfache Passagierung auf CV-1-Zellen unter Benutzung von β-Galactosidase-positiven Plaques für die Selektion.

Rekombinante Vakziniaviren, welche von Plasmid pSC-mGP7 abstammen, wurden vSC-GP7 und solche vom Plasmid pSC-mGP8 wurden vSC-GP8 benannt.

Die Expression von GP wurde erreicht durch Infektion von 1 x 106 Hela Zellen oder von gleichviel RK-13 Zellen mit 10 PFU des vSC-GP7 oder vSC-GP8 pro Zelle.

Die Expression der Genprodukte wurde mit Hilfe der Immunoblot Methode analysiert. Hierzu wurden Lysate von 1,4 x 105 infizierten Hela-Zellen oder RK-13 Zellen auf 10% SDS-PAGE aufgetrennt (wie von Laemmli, Nature 227, 680 (1970) beschrieben) und auf PVDF-Membrane (Fa. Millipore) entsprechend der Halbtrockentechnik aufgetragen. Sekretiertes sGP wurde durch Auftrag von 20 µl des Überstandes (2 ml) von 1 x 10⁶ infizierten Zellen auf das Gel analysiert. Die Immunanalyse erfolgte mit Hilfe eines Maus-anti-EBO-Serums oder eines Pferde-anti-EBO-Serums und einem Kaninchen-anti-Maus- oder anti-Pferdantikörpers als Zweitantikörper, jeweils konjugiert mit Meerrettichperoxidase. Der gebundene Zweitantikörper wurde mit Hilfe der ECL-Technik (Fa. Amersham) analysiert.

In Zellysaten der infizierten Hela-Zellen wie auch der infizierten RK-13 Zellen konnte GP wie auch sGP nachgewiesen werden, wobei nach Infektion mit vSG-GP7 der Anteil von sGP und nach Infektion mit vSC-GP8 der Anteil von GP deutlich größer war.

Endoglycosidase H-Behandlung der Zelllysate und SDS-PAGE Analyse ergab, daß das reife GP ein Molekulargewicht von 125-140 kD aufweist und (im Gegensatz zu "unreifem" GP) resistent ist gegen Spaltung durch Endoglycosidase-H aufgrund der komplexen N-glykosidisch gebundenen Oligosaccharide.

RK-13 Zellen exprimieren ein reifes GP mit einem Molekulargewicht von 140 kD, während Hela-Zellen ein reifes GP von 125 kD exprimieren. Die Unterschiede in der Größe sind auf eine zellspezifische unterschiedliche N-Glykosilierung zurückzuführen. Das 140 kD GP von RK-13 Zellen kornigrierte im SDS-PAGE mit dem GP von Ebolaviren.

Zellen infiziert mit vSC-GP7 wiesen nur geringe Mengen des sGP im Zellysat auf. Dieses hatte ein Molekulargewicht von einheitlich 50 kD. Der überwiegende Anteil von sGP war im Zellüberstand anzutreffen (Verhältnis sekretiertes sGP zu intrazellulärem sGP wie 25:1). Sekretiertes sGP hat ein Molekulargewicht im Bereich von 50-55 kD und ist resistent gegen Endoglycosidase H.

### Auswahl und Reinigung des GP

Als Ligand zur Herstellung des erfindungsgemäßen nicht viralen Vektors wurde das EBO-GP, MG 140 kD, produziert von RK-13 Zellen ausgewählt.

RK-13 Zellen wurden mit 10 PFU des vSC-GP8/Zelle infiziert. 16 bis 18 Stunden nach der Infektion wurden die Zellen geerntet und lysiert. Die Proteine im Lysat wurden aufgetrennt in einer präparativen 8% SDS-PAGE und mit Coomassie brilliant blue gefärbt. Das GP wurde herausgeschnitten, die Gelabschnitte in eine BioTrap (Fa. Schleicher und Schüll) plaziert und diese wiederum in eine horizontale Elektrophoresekammer gelegt. Die Elektroelution erfolgte im Puffer (100 mM Glycin, 20 mM TRIS und 0,01% SDS) für 16 bis 20 Stunden bei 4°C und konstanter Spannung (200 V). Das Eluate wurde aufgefangen und mit Hilfe des Centricon-100 Microconcentrators (Fa. Amicon) konzentriert.

Eine Probe des konzentrierten Eluates wurde auf 10% SDS-PAGE elektrophoretisch aufgetrennt und mit Hilfe der Coomassie brilliant blue Färbung und Immunoblotting auf Reinheit untersucht.

Nachfolgend wurde das GP hochgereinigt mit Hilfe der "reversed phase HPLC" [WP 300 Säule (0,46 x 25 cm), C4,5 µm (Fa. Shandon)] unter Verwendung eines Acetonitrilgradienten (0% bis 100% B in 40 min.; A:0,1% TFA, 10% Acetonitril; B:0,1% TFA; 90% Acetonitril) bei 60°C und mit einer Durchflußgeschwindigkeit von 1 ml/min.

### Beispiel 2

### Herstellung des Fusionsproteins M2 von Influenza A

Die Herstellung des M2-Proteins erfolgt wie von Zebedee et al., J. Virol. 56, 502 (1985); Pinto et al., Cell 69, 517 (1992) und Black et al., J. Gen. Virol. 74, 1673 (1993) im Detail beschrieben.

### Beispiel 3

### Herstellung des kationisierten Trägers auf Proteinbasis (Albumin)

Die Herstellung und Charakterisierung des Trägersystems erfolgt wie von Müller, Dissertation Basel (1994) beschrieben. Die Partikel wurden bezüglich ihrer Größe (Photonenkorrelationsspektroskopie), Oberflächenladung (Zetapotentialmessung) und Morphologie (Rasterelektronenmikroskopie) entsprechend der dem Fachmann bekannten Methoden charakterisiert, beschrieben bei Müller, Dissertation Basel (1994) und Junginger et al., Pharm. Ztg. 25, 9 (1991).

Die Kationisierung des Albumins erfolgte wie von Bergmann et al., Clin. Sci. 67, 35 (1984) und Kumagai et al., J. Biol. Chem. 262, 15214 (1987) im Detail beschrieben. Humanes Serumalbumin wurde nach Aktivierung der Carboxylgruppen mit N-Ethyl-N'-3-(dimethylaminopropyl) carbodiimidhydrochlorid durch kovalente Kopplung von Hexamethylendiamin positiviert. Die Abtrennung nicht umgesetzter Anteile erfolgte mittels Dialyse und Säulenchromatographie. Das Ausmaß der Kationisierung läßt sich durch die Bestimmung des Zetapotentials und mit Hilfe elektrophoretischer Methoden ermitteln.

### 3.1. Herstellung von kationisiertem humanen Serumalbumin (cHSA)

Zu 67 ml einer 2 M Hexamethylendiaminlösung werden 5 ml einer 20 %igen Lösung humanen Setumalbumins zugegeben und ein pH-Wert von 7,8 eingestellt. Der Ansatz wird 30 min bei Raumtemperatur und 100 Upm gerührt und mit 1 g N-Ethyl-N'-3-(dimethyl-aminopropyl)-carbodiimidhydrochlorid versetzt. Nach einer erneuten pH-Kontrolle wird 4 h lang bei Raumtemperatur gerührt und eine Überhitzung durch gelegentliche Inkubationen im Eisbad vermieden. Das Endprodukt wird in einem Amicon-Konzentrator (MWCO 30.000) in fünf Zentrifugationsschritten konzentriert und von niedermolekularen Bestandteilen gereinigt. Der Reinigungsvorgang wird photometrisch (280 nm) und osmometrisch verfolgt. Das gereinigte Derivat wird lyophilisiert und bei 4°C aufbewahrt.

### 3.2. Charakterisierung von cHSA

Die Charakterisierung des kationisierten Albumins erfolgt durch literaturbekannte Elektrophoresetechniken. Die isoelektrische Fokussierung mit homogenen Polyacrylamidgelen (5 % T, 3 % C) und Pharmalyte Carrier Ampholyten im pl-Bereich 3-9 (Phast Gel IEF 3-9) zeigt eine Verschiebung des isoelektrischen Punktes von pl 4 zu pl 7-9. Das Molekulargewicht des Albumins von 67.000 wird durch die Kationisierung nicht verändert, ermittelt durch SDS-PAGE (Phast Gel gradient 10-15, Phast Gel SDS Buffer Strips, nicht reduzierend). Höhermolekulare Aggregate sind mit der gleichen Technik nicht nachweisbar.

Das UV-Absorptionsmaximum des kationisierten Albumins liegt bei 276-278 nm. Das Ausmaß der Kationisierung wird fluorimetrisch durch Umsetzung der primären Aminfunktionen mit Fluorescamin bei 390 nm (Excitation) / 475 nm (Emission) und pH 7 quantifiziert. Ein Umrechungsfaktor von 2,0-2,3 deutet auf eine vollständige Umsetzung hin.

Kationisiertes Albumin läßt sich z.B. im Gemisch mit Plasmiden photometrisch durch den literaturbekannten BCA-Protein Assay im Konzentrationsbereich von 5-10 µg/ml photometrisch bei 562 nm störungsfrei bestimmen.

### 3.3. Funktionsprüfung des cHSA

### a) Herstellung makromolekularer Plasmid/cHSA-Komplexe

22,95 µg CMV-lacZ-Plasmid werden 10 min lang bei Raumtemperatur und 100 Upm in 1,4 ml steriler 0,9 %iger Kochsalzlösung inkubiert. 1 ml einer sterilfiltrierten Lösung des kationisierten humanen Serumalbumin in 0,9 %iger Kochsalzlösung wird mit einer Tropfgeschwindigkeit von einem Tropfen pro Sekunde der Plasmidlösung zugesetzt und 5 min lang bei Raumtemperatur unter Rühren komplexiert.

### b) Charakterisierung der Komplexe

Die zur Neutralisation und Kationisierung des Plasmids notwendige cHSA-Konzentration wird durch Agarosegel-Elektrophorese (1 % Agarosegel, TAE-Puffer pH 7,4, 90 V, 3 h) ermittelt. Nicht gebundene Plasmidanteile werden durch Ethidiumbromid-Interkalation detektiert, Proteinanteile durch eine anschließende Commassie-Färbung. Plasmid-Protein-Komplexe mit positiver Gesamtladung wandern unter den gewählten Bedingungen deutlich sichtbar zur Kathode. Komplexe, hergestellt mit 0,3 ml der 8,3 mg/ml-cHSA-Lösung weisen eine negative, Komplexe, hergestellt mit 0,6 ml, 1 ml, 1,5 ml und 2 ml eine positive Nettoladung auf. Bei keiner der gewählten Konzentrationen treten freie Plasmidanteile auf.

10 minütiges inkubieren in physiologischer Kochsalzlösung schädigt die Plasmide nachweisbar nicht.

Die zur Transfektion geeignete Komplexgröße wird mit Hilfe der Photonenkorrelationspektroskopie (Zetasizer 1, AZ 110, 90°, Wellenlänge 633) bestimmt. Durch Variation
des Inkubationsvolumens
der Art und Ionenstärke des Inkubationsmediums
der cHSA-Konzentration
der Inkubationsdauer des cHSA
der Einspritzgeschwindigkeit der cHSA-Lösung
und des pH-Wert des Inkubationsmediums
wird die Komplexgröße auf 310-360 nm eingestellt. Es entstehen opaleszierende Lösungen.

### c) Transfektion von Zellen mit cHSA/CMV-lacZ-Komplexen

3T3-Zellen werden in DMEM mit 10 % FKS pH 7,1 ohne Zusatz von Antibiotika bei 37°C, 5 % CO₂ und 89 % Feuchtigkeit kultiviert.

### d) Gentransfer

200.000 3T3-Zellen werden in gelatinebeschichtete 3 cm-Petrischalen ausgesät und 24 h lang bis zu einer Konfluenz von ca. 50 % kultiviert. Das Medium wird entfernt, die Zellen 3x mit PBS ohne Calcium und Magnesium pH 7,4 gewaschen und mit 2 ml DMEM ohne FKS oder 1 ml 150 mM NaCl/10 mM HEPES pH 7,4 versetzt. Es wird jeweils mit 0,84 ml des unter 2.1. beschriebenen Plasmid/cHSA-Komplexes, entsprechend 8 µg DNA, verdünnt und bei 37°C 1 h (NaCl/HEPES) bzw. 2 h (DMEM) lang inkubiert.

Zusätzlich werden dem Transfektionsansatz zur Modifikation fusogener und lysosomotroper Eigenschaften verschiedene Agenten wie Glycerol 85 % (sterilisiert, 200 µl, 1,84 ml) zugesetzt. Nach der Inkubation wird das Transfektionsmedium abgesaugt, ersetzt durch DMEM mit FKS und für weitere 48 h inkubiert. Zur Simulation lysosomotroper Effekte wird ein Teil der Ansätze nach der 1- bzw. 2stündigen Transfektionsdauer mit chloroquinhaltigem DMEM (DMEM, 2,5 % FKS, 0,1 mM Chloroquin) für weitere 3 h inkubiert. Danach wird die Chloroquin-Lösung durch DMEM mit 10 % FKS ersetzt.

Zur Kontrolle der Versuchsbedingungen wird eine Transfektion der 3T3-Zellen mit Lipofectamin nach literaturbekannter Methode (10 µl Reagenz, 2 µg DNA) durchgeführt.

Nach 48 h wird das Zellkulturmedium entfernt, die Zellen 1x mit PBS ohne Calcium und Magnesium pH 7,4 gewaschen und 10 min lang mit 0,1 % Glutaraldehyd in PBS fixiert. Überschüssiges Glutaraldehyd wird durch erneutes, zweimaliges Waschen mit PBS entfernt. Transfizierte Zellen werden durch Inkubation mit einer Lösung aus 0,003 M Kaliumferricyanid, 0,003 M Kaliumferrocyanid und 0,08 % X-Gal (stock: 2 % in DMF) in PBS über Nacht bei Raumtemperatur gefärbt und mikroskopisch ausgewertet.

Reine Plasmid / cHSA-Komplexe führen in keinem Fall zu einer Transfektion. Der Zusatz von Glycerol 85 % zum Zellkulturmedium hat bereits nach 1 h das Absterben der Zellen zur Folge. Transfektionsraten entsprechend den Literaturwerten von DEAE-Dextran sind in NaCl/HEPES-gepufferter Lösung nach Chloroquinschock zu beobachten. In DMEM ist der Gentransfer mit der gleichen Nachbehandlung wesentlich schwächer ausgeprägt.

**Tabelle 1**

| Übersicht über die Charakterisierung des kationisierten humanen Serumalbumins | | |
|---|---|---|
| | Methode | Ergebnis |
| Löslichkeit | - | gut in Wasser und physiologischen Medien |
| pl | isoelektrische Fokussierung | pl 7-9 |
| Molekulargewicht | SDS-PAGE | 67.000 |
| höhermolekulare Aggregate | SDS-PAGE | keine Aggregate nachweisbar |
| Absorptionsmaximum | UV-Spektroskopie | 276-278 nm |
| Ausmaß der Kationisierung | Fluorimetrie (Fluorescamin) | theoret.: 2,18 prakt.: 2,0-2,3 |
| Bestimmung der Gesamtladung | Agarosegel-Elektrophorese | positive Ladung |
| Gehaltsbestimmung | BCA Protein Assay | - |

**Tabelle 2**

| Validierung der Bedingungen der Komplexbildung |
|---|
| Inkubationsvolumen: |
| • 110 µl |
| • 1,4 ml |

| Art und Ionenstärke des Inkubationsmediums: |
|---|
| • bidestilliertes Wasser |
| • PBS ohne Calcium und Magnesium pH 7,4 |
| • physiologische Kochsalzlösung |
| • 150 mM NaCl, 10 mM HEPES pH 7,4 |
| • DMEM ohne Serum pH 7,4 |

| cHSA-Konzentration: |
|---|
| getestete Konzentrationen: |
| • 0,1 µg, 1µg, 10 µg, 100 µg, 0,5 mg, 1 mg, 1,5 mg, 2 mg, 2,5 mg, 3 mg, 5 mg, 10 mg jeweils pro ml |

| ausgewählte Konzentrationen: |
|---|
| • 2,49 mg, 5,16 mg, 8,3 mg, 12,45 mg, 16,6 mg jeweils pro ml |

| Inkubationsdauer |
|---|
| • 5, 30, 60, 120, 180 min |

| pH-Wert des Inkubationsmediums: |
|---|
| • pH 4,0 |
| • pH 7,4 |
| • pH 9 |

| Einspritzgeschwindigkeit |
|---|
| • 1 Tropfen/sec |
| • sofort vollständige cHSA-Zugabe |

### Beispiel 4

### Herstellung des Plasmids

Der humane Endothelin-1 Promoter (Position 〈 -170 bis 〉 -10; Wilson et al., Mol. Cell Biol. 10, 4654 (1990)) oder eine um die TATA-Box verkürzte Variante (Position 〈 -170 bis 〉 -40) werden an ihrem 3' Ende mit dem 5'-Terminus des CDE-CHR-Inr Moduls (Position 〈 -20 bis 〉 +121) des humanen cdc25C-Gens (UK 950.6466.3) verknüpft. Die Verknüpfung erfolgt mit Hilfe von dem Fachmann bekannten und käuflichen Enzymen.

Die so hergestellte chimäre Endothelin-1 Promotermodul-Transkriptionseinheit wurde an ihrem 3' Ende mit dem 5'-Terminus einer DNA, die den kompletten kodierenden Bereich der humanen β-Glucuronidase enthält (Position 〈 27 bis 〉 1982; Oshima et al., PNAS USA 84, 685 (1987)), verknüpft. Diese DNA enthält auch die für eine Sekretion notwendige Signalsequenz (22 N-terminale Aminosäuren). Zur Erleichterung der zellulären Ausschleusung wurde diese Signalsequenz ausgetauscht gegen die Signalsequenz des Immunglobulins (Position 〈 63 bis 〉 107; Riechmann et al., Nature 332, 323 (1988)). Transkriptionskontrolleinheiten und die DNA für β-Glucuronidase wurden in pUC18/19 oder Bluescript-abgeleiteten Plasmidvektoren mit Hilfe von dem Fachmann bekannten und käuflichen Enzymen einkloniert.

### Beispiel 5

### Komplexierung des kationisierten Trägers mit dem Plasmid

Die Komplexierung des Trägers mit dem Plasmid erfolgt durch Inkubation der beiden Komponenten in einem geeigneten Mischungsverhältnis. Das Ausmaß der Assoziation wurde über die Veränderung des Zetapotentials ermittelt.

6,5 ml einer 20%igen Lösung des kationischen HSA (hergestellt wie unter c) beschrieben und ausgewählt aus einem Bereich von 5-35%) wurden im Verhältnis 1:2 (ausgewählt aus einem Bereich von 1:1 bis 1:10) mit der Plasmidlösung versetzt. Die äußere Phase, bestehend aus 93,5 ml Dichlormethan/Methanol (9:1) mit 0,5% Klucel GF wurde 30 min lang auf 20° C temperiert und die Albuminlösung der mit einem Durchsatz von 500 ml/min zirkulierenden, organischen Phase zugemischt. Die Emulsion wurde 15 min lang mit 65 Watt gepulst beschallt.

Zur Vernetzung wurden 6,6 mmol Glutaraldehyd in Methylenchlorid zum Ansatz dazugegeben und 80-100 min lang bei 2200 rpm bei Raumtemperatur gerührt. Zur Reinigung wurden die Partikel mehrmals gewaschen und abzentrifugiert.

### Beispiel 6

### Einführung von lipophilen Gruppen

Die Einführung lipophiler Gruppen erfolgte durch Acylierung unter dem Fachmann bekannten Bedingungen. Das Carbonsäurederivat reagiert dabei mit den primären Aminogruppen des Albumins nach dem bekannten Additions-Eliminierungs-Mechanismus der Acylierung zum Carbonsäureamid.

0,1 g Ölsäurechlorid wurde in 5-10 ml wasserfreiem Dioxan gelöst und tropfenweise mit einer Suspension der Partikel (hergestellt wie unter c) beschrieben) in Dioxan im Verhältnis 1:4 (ausgewählt aus einem Bereich von 1:1 - 1:10) versetzt und kräftig geschüttelt. Nach Zugabe eines Überschusses wäßriger Ammoniaklösung wurde der Ansatz 10 min lang gerührt und mit verdünnter Salzsäure schwach angesäuert. Die Partikel wurden durch Zentrifugation abgetrennt und mit Wasser neutral gewaschen.

### Beispiel 7

### Konjugation von Liganden und Fusionsprotein an den Träger

Die Verknüpfung von Liganden und Fusionsproteinen mit dem Trägersystem erfolgt durch kovalente Kopplung nach der SPDP-Methode, wie bei Khawli et al., Int. J. Rad. Appl. Instrum. B 19, 289 (1992) und Candiani et al., Cancer Res. 62, 623 (1992) beschrieben. Primäre Aminofunktionen der Lysinreste des Albumins reagieren dabei mit SPDP zu disulfidhaltigen Derivaten. Diese lassen sich mit Sulfhydrylgruppen der Liganden und Fusionsproteine unter dem Fachmann bekannten Bedingungen kovalent verbinden.

200 nmol SPDP-Reagenz in 99,5%igem Ethanol wurden mit 74 nmol kationisiertem, lipophilisiertem HSA-Partikeln (hergestellt wie unter f) beschrieben) in PBS 30 min lang bei Raumtemperatur inkubiert. Zur Konjugation wurde der Ansatz mit einer Lösung des Ebolavirus Glykoprotein GP (hergestellt wie unter a) beschrieben) und des Fusionsproteins M2 von Influenza (hergestellt wie unter b) beschrieben) im Verhältnis 1:1 (ausgewählt aus einem Bereich von 1:1 - 1:10) versetzt und über Nacht unter Rühren bei Raumtemperatur inkubiert. Nicht gebundene Anteile wurden abzentrifugiert.

### Beispiel 8

### Wirksamkeit des zielzellspezifischen Vektors

Der zielzellspezifische Vektor, hergestellt wie in den vorhergehenden Beispielen beschrieben, wird nach systemischer, bevorzugt intravenöser oder intraarterieller Gabe durch die gewebespezifischen Liganden bevorzugt an Endothelzellen gebunden. Nach erfolgter Aufnahme in die Endosomen erfolgt eine durch das Fusionsprotein vermittelte Penetration in das Zytoplasma. Durch die gewebespezifische Promotorsequenz und das zellzyklusregulierte Promotormodul erfolgt eine vorwiegende Expression des Genes in proliferierende Endothelzellen. Durch diese Gene wird von diesen proliferierenden Endothelzellen β-Glucuronidase ausgeschieden, die pharmakologisch inaktive β-Glucuronidide (Prodrugs) in aktive Substanzen spaltet. Diese aktive Substanz kann beispielsweise antiproliferativ oder zytostatisch wirken. Hierdurch kommt es zur Hemmung der Endothelzellproliferation und zur Hemmung des Wachstums eines benachbarten Tumors oder einer benachbarten Entzündungsreaktion. Da durch den erfindungsgemäßen Wirkstoff die Entstehung der antiproliferativen oder zytostatischen Substanz auf den Ort der durch den Tumor oder durch die Entzündung verursachten Angiogenese beschränkt ist, ist die Verträglichkeit des Wirkstoffes gut.

Durch die Wahl des (nichtviralen) Trägers für das ausgewählte Gen sind keine Risiken durch Mutationen der Gene des Patienten, durch Aktivierung im Genom integrierter ruhender Viren oder durch Rekombination mit Wildviren zu erwarten.

## Patentansprüche

1. Zielzellspezifischer Vektor für die Einschleusung wenigstens eines Genes in Zellen eines Organismus umfassend folgende Komponenten:
a) einen nicht-viralen Träger für das einzuschleusende Gen,
b) einen Liganden, der spezifisch an die gewünschte Zielzelle binden kann,
c) ein Fusionsprotein oder ein fusiogenes Peptid für die Penetration des Vektors in das Zytoplasma der Zielzelle und
d) das einzuführende Gen.

2. Vektor nach Anspruch 1, dadurch gekennzeichnet, daß die einzelnen Komponenten des zielzellspezifischen Vektors miteinander kovalent und/oder durch adsorptive Bindung verbunden sind.

3. Vektor nach einem der Ansprüche 1 oder 2, dadurch gekennzeichnet, daß der nicht-virale Träger (a) für das Gen ausgewählt ist aus der Gruppe umfassend Proteine, Polypeptide, Polysaccharide, Phospholipide, kationische Lipide, Glykoproteine, Lipoproteine oder Lipopolyamine.

4. Vektor nach Anspruch 3, dadurch gekennzeichnet, daß der nicht-virale Träger (a) durch Einführung positiv geladener Seitengruppen kationisiert ist, wobei die Bindung zwischen nicht-viralem Träger und positiv geladener Seitenkette durch adsorptive oder kovalente Bindung bewirkt wird.

5. Vektor nach einem der vorhergehenden Ansprüche, dadurch gekennzeichnet, daß der nicht-virale Träger (a) durch adsorptive oder kovalente Bindung verbundene lipophile Seitengruppen aufweist, wodurch der Träger amphiphile Eigenschaften erhält.

6. Vektor nach einem der vorhergehenden Ansprüche, dadurch gekennzeichnet, daß der nicht-virale Träger (a) Albumin oder Xylan ist.

7. Vektor nach einem der vorhergehenden Ansprüche, dadurch gekennzeichnet, daß der Ligand (b) spezifisch an die äußere Membran tierischer oder menschlicher Zellen binden kann.

8. Vektor nach Anspruch 7, dadurch gekennzeichnet, daß der Ligand (b) spezifisch an Endothelzellen binden kann und ausgewählt ist aus der Gruppe bestehend aus monoklonalen Antikörpern oder deren Fragmenten, die spezifisch sind für Endothelzellen, Kinine oder Analoge oder Homologe hiervon, endständig Mannose tragende Glykoproteine, Glykolipide oder Polysaccharide, Zytokine, Wachstumsfaktoren, Adhäsionsmoleküle oder Glykoproteine aus der Hülle von Viren, die einen Tropismus für Endothelzellen haben.

9. Vektor nach Anspruch 7, dadurch gekennzeichnet, daß der Ligand (b) spezifisch an glatte Muskelzellen binden kann und ausgewählt ist aus der Gruppe umfassend monoklonale Antikörper oder deren Fragmente, die spezifisch binden an Aktin, Zellmembranrezeptoren, Wachstumsfaktoren oder Glykoproteine aus der Hülle von Viren, die einen Tropismus für glatte Muskelzellen haben.

10. Vektor nach Anspruch 7, dadurch gekennzeichnet, daß der Ligand (b) spezifisch binden kann an Makrophagen und/oder Lymphozyten und ausgewählt ist aus der Gruppe umfassend monoklonale Antikörper, die spezifisch binden an Membranantigene auf Makrophagen und/oder Lymphozyten, intakte Immunglobuline oder Fc-Fragmente von polyklonalen oder monoklonalen Antikörpern, die spezifisch binden an Membranantigene auf Makrophagen und/oder Lymphozyten, Zytokine, Wachstumsfaktoren, Mannose-endständig tragende Peptide, Proteine, Lipide oder Polysaccharide, Glykoproteine aus der Hülle von Viren, das HEF-Protein vom Influenza C-Virus mit Mutation in der Nukleotidposition 872 oder HEF-Spaltprodukte des Influenza C-Virus enthaltend die katalytische Triade Serin-71, Histidin 368 oder -369 und Asparaginsäure 261.

11. Vektor nach Anspruch 7, dadurch gekennzeichnet, daß der Ligand (b) spezifisch binden kann an Gliazellen und ausgewählt ist aus der Gruppe umfassend Antikörper und Antikörperfragmente, die spezifisch binden an Membranstrukturen von Gliazellen, Adhäsionsmoleküle, endständig Mannose tragende Peptide, Proteine, Lipide oder Polysaccharide, Wachstumsfaktoren, Glykoproteine aus der Hülle von Viren, die einen Tropismus für Gliazellen haben.

12. Vektor nach Anspruch 7, dadurch gekennzeichnet, daß der Ligand (b) spezifisch binden kann an blutbildende Zellen und ausgewählt ist aus der Gruppe umfassend Antikörper oder Antikörperfragmente, die spezifisch sind für einen Rezeptor des Stem cell factors, IL-1 (Rezeptortyp I oder II), IL-3 (Rezeptortyp a oder β), IL-6 oder GM-CSF sowie intakte Immunglobuline oder Fc-Fragmente, die diese Spezifität aufweisen und Wachstumsfaktoren sowie deren Fragmente, die an die zugehörigen Rezeptoren binden.

13. Vektor nach Anspruch 7, dadurch gekennzeichnet, daß der Ligand (b) spezifisch binden kann an Leukämiezellen oder Tumorzellen und ausgewählt ist aus der Gruppe umfassend Steroidhormone, Peptidhormone, Antikörper, Antikörperfragmente, Immunglobuline oder Fc-Fragmente, die spezifisch binden an Membranstrukturen auf Leukämiezellen wie CD13, CD14, CD15, CD33, CAMAL, Sialosyl-Le, CD5, CD1e, CD23, M38, IL-2-Rezeptoren, T-Zell-Rezeptoren, CALLA oder CD19 sowie Wachstumsfaktoren oder davon abstammende Fragmente oder Retinoide.

14. Vektor nach Anspruch 7, dadurch gekennzeichnet, daß der Ligand (b) spezifisch an virusinfizierte Zellen binden kann und ausgewählt ist aus der Gruppe umfassend Antikörper, Antikörperfragmente, intakte Immunglobuline oder Fc-Fragmente, die spezifisch sind für ein Virusantigen, das nach Infektion durch das Virus auf der Zellmembran der infizierten Zelle exprimiert wird.

15. Vektor nach Anspruch 7, dadurch gekennzeichnet, daß der Ligand (b) spezifisch binden kann an Bronchialepithelzellen, sinusoidale Zellen der Leber oder Leberzellen und ausgewählt ist aus der Gruppe umfassend Transferrin, Asialoglycoproteine, wie Asialoorosomucoid, Neoglycoprotein oder Galactose, Insulin, endständig Mannose tragende Peptide, Proteine, Lipide oder Polysaccharide, intakte Immunglobuline oder Fc-Fragmente, die spezifisch an diese Zielzellen binden und Glykoproteine aus der Hülle von Viren, die spezifisch an diese Zielzellen binden.

16. Vektor nach einem der vorhergehenden Ansprüche, dadurch gekennzeichnet, daß das Fusionsprotein (c) ausgewählt ist aus dem Haemagglutinin von Influenza A- oder B-Viren, der HA2-Komponente des Haernagglutinins von Influenza A- oder B-Viren sowie Peptidanaloga hiervon, das M2-Protein von Influenza A-Viren, das HEF-Protein von Influenza C-Viren, Transmembranproteine von Filoviren, Transmembranglykoproteine von Tollwutvirus, Vesicular Stomatitis Virus, Semliki Forest-Virus, Tickborn Encephalitis-Virus, Fusionsproteine des HIV-Virus, des Sendai-Virus, des respiratorischen syncytialen Virus sowie Fragmente dieser viralen Fusionsproteine oder der Transmembranglykoproteine, bei denen die Transmembranregion entfernt wurde.

17. Vektor nach einem der vorhergehenden Ansprüche, dadurch gekennzeichnet, daß das einzuführende Gen (d) in Form eines Plasmids vorliegt.

18. Arzneimittel, dadurch gekennzeichnet, daß es einen Vektor nach einem der vorhergehenden Ansprüche aufweist.

19. Verwendung eines Vektors nach einem der Ansprüche 1 bis 7 zur Herstellung eines Arzneimittels für die intravenöse, intraarterielle, intraportale, intrakraniale, intrapleurale, intraperitoneale oder lokale Einführung eines gewünschten Gens in bestimmte Zielzellen.
